# EUROPÄISCHE PATENTSCHRIFT

(11) **EP 2 155 376 B1**
(45) Veröffentlichungstag und Bekanntmachung des Hinweises auf die Patenterteilung: **11.07.2012**
(21) Anmeldenummer: 08760298.3
(22) Anmeldetag: 30.05.2008
(51) Int. Cl.: B01J 8/06

(54) **VERFAHREN DER WIEDERBESCHICKUNG DER REAKTIONSROHRE EINES ROHRBÜNDELREAKTORS MIT EINEM NEUEN KATALYSATORFESTBETT**
METHOD OF RECHARGING THE REACTION TUBE OF A TUBE-BUNDLE REACTOR WITH A NEW CATALYST FIXED BED
PROCÉDÉ DESTINÉ À RECHARGER LES TUBES DE RÉACTION D'UN RÉACTEUR À FAISCEAU DE TUBES AVEC UN NOUVEAU LIT CATALYTIQUE SOLIDE

(30) Priorität: 01.06.2007 DE 102007025869; 01.06.2007 US 941385 P
(43) Veröffentlichungstag der Anmeldung: 24.02.2010
(73) Patentinhaber: BASF SE, 67056 Ludwigshafen (DE)
(72) Erfinder: SCHLIEPHAKE, Volker, 67105 Schifferstadt (DE); BOTT, Klaus, 67071 Ludwigshafen (DE); BECHER, Rolf-Dieter, 68199 Mannheim (DE); MÜLLER-ENGEL, Klaus Joachim, 76297 Stutensee (DE); PETZOLDT, Jochen, 67273 Weisenheim am Berg (DE); CREMER, Ulrich, 68161 Mannheim (DE); RAICHLE, Andreas, 67063 Ludwigshafen (DE)
(86) Internationale Anmeldenummer: PCT/EP2008/056711
(87) Internationale Veröffentlichungsnummer: WO 2008/145739

(56) Entgegenhaltungen:
- WO-A-98/02239
- WO-A-2005/082522
- DE-A1- 10 353 617
- US-A- 4 701 101

## Beschreibung

Vorliegende Erfindung betrifft ein Verfahren der Wiederbeschickung der Reaktionsrohre eines Rohrbündelreaktors mit einem neuen Katalysatorfestbett zum Zweck der Durchführung einer neuen heterogen katalysierten partiellen Gasphasenoxidation einer organischen Ausgangsverbindung in dem neuen Katalysatorfestbett, bei dem vor der Wiederbeschickung der Reaktionsrohre des Rohrbündelreaktors mit dem neuen Katalysatorfestbett in den selben Reaktionsrohren eine heterogen katalysierte partielle Vorgängergasphasenoxidation einer organischen Verbindung in einem in diesen Reaktionsrohren befindlichem Katalysatorfestbett, das wenigstens Katalysatorformkörper umfasste, deren Aktivmasse ein das Element Mo in oxidiertem Zustand enthaltendes Multielementoxid ist, unter Erzeugung eines Wasserdampf enthaltenden Produktgasgemischs durchgeführt worden und das Vorgängerkatalysatorfestbett nach Beendigung dieser Partialoxidation aus den Reaktionsrohren entnommen worden ist.

Verfahren der heterogen katalysierten partiellen Gasphasenoxidation von organischen Verbindungen in in den Reaktionsrohren von Rohrbündelreaktoren befindlichen Katalysatorfestbetten, die wenigstens Katalysatorformkörper umfassen, deren Aktivmasse ein das Element Mo in oxidiertem Zustand enthaltendes Multielementoxid (z. B. Multimetalloxid) ist, sind zur Herstellung von Industriechemikalien wie z. B. Acrylsäure, Acrolein, Methacrylsäure, Methacrolein, Acrylnitril und Methacrylnitril bekannt (vgl. z. B. WO 2005/030393, DE-A 10 2007 005, DE-A 10 2007 004 961, Research Disclosure RD 2005/497012, EP-A 467144, EP-A 714700, US 2006/0205978 und DE-A 10 2004 025 445). Bei diesen heterogen katalysierten partiellen Gasphasenoxidationen enthält das Produktgasgemisch normalerweise Wasserdampf, der einerseits in der Regel als Nebenprodukt der Partialoxidation gebildet und andererseits dem Reaktionsgaseingangsgemisch vielfach bereits als inertes Verdünnungsgas zugesetzt wird.

Bei einem Rohrbündelreaktor handelt es sich dabei um einen Apparat, der ein vertikal angeordnetes Bündel von Reaktionsrohren umfasst, welches von einem Reaktormantel umschlossen wird, wobei beide Enden der einzelnen Reaktionsrohre offen sind und jedes Reaktionsrohr mit seinem oberen Ende in eine Durchgangsöffnung eines oben in den Reaktormantel eingedichteten oberen Rohrbodens und mit seinem unteren Ende in eine Durchgangsöffnung eines unten in den Reaktormantel eingedichteten unteren Rohrbodens eingedichtet ausläuft, wobei das Äußere der Reaktionsrohre, der obere und der untere Rohrboden, und der Reaktormantel gemeinsam den Reaktionsrohrumgebungsraum abgrenzen, sowie jeder der beiden Rohrböden von einer wenigstens eine Öffnung aufweisenden Reaktorhaube überspannt wird. Bei der Durchführung einer heterogen katalysierten partiellen Gasphasenoxidation in einem solchen Rohrbündelreaktor sind dessen Reaktionsrohre mit einem Katalysatorfestbett beschickt (ist in dessen Reaktionsrohren ein Katalysatorfestbett eingefüllt; befindet sich in dessen Reaktionsrohren ein Katalysatorfestbett) und über die wenigstens eine Öffnung in einer der beiden Reaktorhauben wird ein die partiell zu oxidierende organische Verbindung und molekularen Sauerstoff enthaltendes Reaktionsgaseingangsgemisch zu- und über die wenigstens eine Öffnung der anderen Reaktorhaube das durch partielle Gasphasenoxidation der partiell zu oxidierenden organischen Verbindung zum gewünschten Zielprodukt beim Durchströmen des in den Reaktionsrohren befindlichen Katalysatorfestbetts resultierendes Zielprodukt enthaltendes Produktgasgemisch abgeführt, während auf der Mantelseite des Rohrbündelreaktors um die Reaktionsrohre wenigstens ein (in der Regel flüssiges) Wärmeaustauschmittel geführt wird. Normalerweise wird im Fall der Verwendung von wenigstens einem flüssigen Wärmeaustauschmittel das selbige so um die Reaktionsrohre geführt, dass jede der beiden einander zugewandten Oberflächen der beiden Rohrböden von flüssigem Wärmeaustauschmittel benetzt wird. Das wenigstens eine (z. B. flüssige) Wärmeaustauschmittel wird üblicherweise mit einer Temperatur T_{w}^{ein} in den Reaktionsrohrumgebungsraum hinein- und mit der Temperatur T_{w}^{aus} wieder aus dem Reaktionsrohrumgebungsraum herausgeführt, wobei T_{w}^{aus} ≥ T_{w}^{ein} gilt. Grundsätzlich kann das wenigstens eine Wärmeaustauschmittel auch gasförmig oder im Siedezustand befindlich durch den Reaktionsrohrumgebungsraum geführt werden. Beispiele für derartige Rohrbündelreaktoren und in selbigen durchgeführte heterogen katalysierte Partialoxidationen offenbaren z. B. die EP-A 700893, die DE-A 4431949, die WO 03/057653, die EP-A 1695954, die WO 03/055835, die WO 03/059857, die WO 03/076373, die DE 69915952 T2, die DE-A 10 2004 018267, die DE 20 2006 014 116 U1 und das Deutsche Aktenzeichen 102007019597.6, sowie der in den vorgenannten Schriften zitierte Stand der Technik.

In der Regel sind die Bauteile des Rohrbündelreaktors aus Stahl gefertigt. Dabei kommt als Fertigungsstahl sowohl Edelstahl (z. B. der DIN Werkstoffnummer 1.4541 oder der Werkstoff 1.4571) als auch Schwarzstahl bzw. ferritischer Stahl (z. B. die DIN-Werkstoffe 1.0481, 1.0315 oder der Werkstoff 1.0425) in Betracht. Häufig sind alle Bauteile des Rohrbündelreaktors aus der gleichen Stahlsorte gefertigt. Vielfach sind die Reaktorhauben aus ferritischem Stahl gefertigt und auf ihrer Innenseite mit Edelstahl plattiert. Teilweise ist der Reaktormantel auch aus einer anderen Stahlsorte als die übrigen Teile des Rohrbündelreaktors gefertigt, da zu seiner Herstellung gewalzter Stahl verwendet werden kann.

Der durch das Äußere der Reaktionsrohre, die beiden Rohrböden und den Reaktormantel zusammen begrenzte Raum, in welchem das wenigstens eine (in der Regel flüssige) Wärmeaustauschmittel geführt wird, soll in dieser Schrift als Reaktionsrohrumgebungsraum bezeichnet werden.
In einfachster Weise wird im Reaktionsrohrumgebungsraum nur ein (vorzugsweise flüssiges) Wärmeaustauschmittel geführt (eine solche Verfahrensweise wird auch als Einzonenfahrweise im Einzonenrohrbündelreaktor bezeichnet). Es wird dem Reaktionsrohrumgebungsraum üblicherweise an seinem oberen oder an seinem unteren Ende mit seiner Eintrittstemperatur T_{w}^{ein} durch Öffnungen im Reaktormantel zu- und am entgegengesetzten Ende mit einer Austrittstemperatur T_{w}^{aus} durch Öffnungen im Reaktormantel wieder aus dem Reaktionsrohrumgebungsraum herausgeführt.

Durch die Exothermie der Gasphasenpartialoxidation bedingt gilt während der Durchführung der Partialoxidation T_{w}^{aus} ≥ T_{w}^{ein} (die Gleichheit bezieht sich auf den Fall einer Siedekühlung). Mit Hilfe eines Wärmeaustauschers wird üblicherweise einer Teil- oder der Gesamtmenge des aus dem Reaktionsrohrumgebungsraum herausgeführten (vorzugsweise flüssigen) Wärmeaustauschmittels Wärme entzogen, bevor es mit der Temperatur T_{w}^{ein} dem Reaktionsrohrumgebungsraum wieder zugeführt wird.
Im Reaktionsrohrumgebungsraum kann das (vorzugsweise flüssige) Wärmeaustauschmittel grundsätzlich im einfachen Gleich- oder Gegenstrom zum in den Reaktionsrohren strömenden Reaktionsgasgemisch um die Reaktionsrohre geführt werden. Es kann aber auch mit Hilfe entsprechender Umlenkscheiben mäanderförmig um die Reaktionsrohre geführt werden, so dass lediglich über den gesamten Reaktionsrohrumgebungsraum ein Gleich- oder Gegenstrom zur Strömungsrichtung des Reaktionsgasgemischs in den Reaktionsrohren besteht. Ist das verwendete Wärmeaustauschmittel unter den Einsatzbedingungen flüssig, sollte es anwendungstechnisch zweckmäßig einen Schmelzpunkt im Bereich von 0 bis oder von 50 bis 250 °C, vorzugsweise von 120 bis 200 °C aufweisen.

Als solche flüssigen Wärmeaustauschmittel kommen z. B. Schmelzen von Salzen wie Kaliumnitrat, Kaliumnitrit, Natriumnitrit und/oder Natriumnitrat sowie Schmelzen von Metallen wie Kalium, Natrium, Quecksilber und Legierungen verschiedener Metalle in Betracht. Aber auch ionische Flüssigkeiten (in denen wenigstens eines der entgegengesetzt geladenen Ionen wenigstens ein Kohlenstoffatom enthält) oder Wärmeträgeröle (z. B. hochsiedende organische Lösungsmittel wie Mischungen aus Diphyl^{®} und Dimethylphthalat) sind einsetzbar. Als gasförmige Wärmeaustauschmittel kommen z.B. unter erhöhtem Druck befindlicher Wasserdampf oder auch Rauchgase in Betracht. Siedekühlung kann z. B. auch mit unter Druck befindlichem Siedewasser vorgenommen werden.

Zur Verbesserung der Selektivität der Zielproduktbildung kann man die heterogen katalysierte partielle Gasphasenoxidation einer organischen Verbindung aber auch als Mehrzonenfahrweise (z. B. Zweizonenfahrweise) in einem Mehrzonenrohrbündelreaktor (z. B. in einem Zweizonenrohrbündelreaktor) ausführen. In diesem Fall werden im Reaktionsrohrumgebungsraum (z. B. zwei) voneinander im wesentlichen räumlich getrennte (vorzugsweise flüssige) Wärmeaustauschmittel (die normalerweise von der selben Sorte sind) geführt (diese können z. B. durch im Reaktionsrohrumgebungsraum eingezogene und für die Reaktionsrohre entsprechende Durchtrittsöffnungen aufweisende Trennrohrböden separiert sein).
Der Reaktionsrohrlängsabschnitt, über den sich das jeweilige (vorzugsweise flüssige) Wärmeaustauschmittel erstreckt, repräsentiert eine Temperatur- bzw. Reaktionszone (der Einzonenrohrbündelreaktor weist in entsprechender Weise nur eine Reaktionszone auf).
Innerhalb der jeweiligen Temperaturzone kann das (vorzugsweise flüssige) Wärmeaustauschmittel wie bei der Einzonenfahrweise geführt werden (auch relativ zur Strömungsrichtung des Reaktionsgasgemischs). Für den Unterschied zwischen T_{w}^{aus} und T_{w}^{ein} gilt die einzelne Temperaturzone betreffend das bezüglich der Einzonenfahrweise Gesagte in im wesentlichen gleicher Weise.

Eine graphische Unterscheidung zwischen einer Einzonen- und einer Zweizonenfahrweise (zwischen einem Einzonenrohrbündelreaktor und einem Zweizonenrohrbündelreaktor) zeigen schematisch z. B. die Figuren des Deutschen Aktenzeichens 102007019597.6 sowie die Figuren der EP-A 1695954. Mehrzonenfahrweisen sind im übrigen z. B. beschrieben in den Schriften EP-A 1734030, DE-A 10313214, DE-A 10313219, DE-A 10313211, DE-A 10313208 sowie in dem in diesen Schriften zitierten Stand der Technik. Sie sind vor allem dann vorteilhaft, wenn eine hohe Belastung des Katalysatorfestbetts mit der partiell zu oxidierenden organischen Verbindung gewählt wird. Unter der Belastung des Katalysatorfestbetts mit Reaktionsgasgemisch oder mit einer Reaktionsgasgemischkomponente wird die Menge an Reaktionsgasgemisch bzw. Reaktionsgasgemischkomponente in Normlitern (NI; das Volumen, das die entsprechende Menge rechnerisch bei 0 °C und 1 atm gasförmig einnehmen würde) verstanden, die pro Stunde durch einen Liter Katalysatorfestbett (reine Inertschüttungen werden nicht miteinbezogen) geführt wird.

Das Reaktionsgaseingangsgemisch (bzw. auch Reaktionsgaseintrittsgemisch) selbst kann bei den unterschiedlichen Verfahrensweisen im Rohrbündelreaktor in den Reaktionsrohren sowohl von oben nach unten als auch von unten nach oben geführt werden (d. h., die wenigstens eine Zufuhröffnung kann sich entweder in der oberen oder in der unteren Reaktorhaube befinden). Das gleiche trifft auf die Führung des (vorzugsweise flüssigen) Wärmeaustauschmittels zu.

Das Reaktionsgaseingangsgemisch kann bei seinem Eintritt in die Reaktionsrohre grundsätzlich auf die Temperatur des auf der entsprechenden Rohrbodenunterseite strömenden Wärmeaustauschmittels vorerwärmt sein.

Die Temperatur des Reaktionsgaseintrittsgemischs kann sich bei dessen Eintritt in die Reaktionsrohre aber auch unterhalb dieser Temperatur des Wärmeaustauschmittels befinden. Dies ist dann sinnvoll, wenn die Reaktionsrohre in Strömungsrichtung des Reaktionsgasgemischs zunächst mit einem Längsabschnitt aus gegenüber der Partialoxidation inerten Formkörpern beschickt sind, bevor der katalytisch aktive (wirksame) Abschnitt des Katalysatorfestbetts mit katalytisch wirksame Aktivmasse aufweisenden Formkörpern beginnt. Beim Durchströmen dieses inerten Abschnitts kann sich das Reaktionsgaseintrittsgemisch dann auf die Temperatur des den entsprechenden katalytisch aktiven Reaktionsrohrabschnitt umströmenden Wärmeaustauschmittels erwärmen. Prinzipiell kann das Reaktionsgaseingangsgemisch (das Produktgasgemisch) über mehr als eine in der entsprechenden Reaktorhaube befindliche Zufuhröffnung (Abfuhröffnung) zugeführt (abgeführt) werden. In der Regel erfolgen aber sowohl die Zufuhr des Reaktionsgaseintrittsgemischs als auch die Abfuhr des Produktgasgemischs jeweils über nur eine Öffnung in der entsprechenden Reaktorhaube.

Häufig kann eine heterogen katalysierte partielle Gasphasenoxidation einer organischen Verbindung räumlich unmittelbar einer heterogen katalysierten partiellen Gasphasenoxidation einer anderen organischen Verbindung nachgeschaltet (in diesem Fall ist das Zielprodukt der vorausgehenden Partialoxidation normalerweise die partiell zu oxidierende organische Verbindung in der nachgeschalteten Partialoxidation) oder vorgeschaltet erfolgen. Insbesondere in diesen Fällen kann die zuführende bzw. abführende Reaktorhaube zu einer zylindrischen Rohröffnung reduziert (als zylindrische Rohröffnung ausgeführt) sein, die z. B. einen zylindrischen Übergang zu einem Nachkühler bilden kann (vgl. z. B. DE-A 10 2004 018267 und Deutsches Aktenzeichen 102007019597.6).

Selbstredend können auch zwei heterogen katalysierte partielle Gasphasenoxidationen, die zwei aufeinanderfolgende Gasphasenpartialoxidationsschritte abbilden, unmittelbar aufeinanderfolgend in den Reaktionszonen eines Mehrzonenrohrbündelreaktors (z. B. in einem Zweizonenrohrbündelreaktor) durchgeführt werden, wobei sich dabei die Beschickung des Katalysatorfestbetts in den Reaktionsrohren des Mehrzonenrohrbündelreaktors beim Übergang vom einen Reaktionsschritt zum nachfolgenden Reaktionsschritt normalerweise in entsprechender Weise ändert (vgl. z. B. die Durchführung mehrstufiger heterogen katalysierter partieller Gasphasenoxidationen im sogenannten "single reactor" gemäß EP-A 1388533, US-A 6069271, EP-A 990636, US-A 2006/0161019 und EP-A 1106598). Beispiele für die Durchführung solcher mehrstufiger heterogen katalysierter partieller Gasphasenoxidationen im Mehrzonenrohrbündelreaktor (z. B. Zweizonenrohrbündelreaktor) sind die heterogen katalysierte partielle Gasphasenoxidation von Propylen zu Acrylsäure sowie von iso-Buten zu Methacrylsäure.

Es ist nun allgemein bekannt, dass heterogen katalysierte partielle Gasphasenoxidationen mit Vorteil in solchen Rohrbündelreaktoren durchgeführt werden, deren Reaktionsrohre im Rahmen des machbaren zum einen möglichst einheitlich gefertigt (vgl. z. B. WO 03/059857, DE-A 20 2006 014 116 U1 und EP-A 1471046) und zum anderen möglichst einheitlich mit dem selben Katalysatorfestbett beschickt sind (vgl. z. B. US-A 4701101, EP-A 1466883, WO 03/057653, US-A 2006/245992 und US-A 2002/136678). Gemäß der Lehre der JP-A 2006-142288 soll die Reaktionsrohrinnenoberfläche darüber hinaus eine möglichst niedrige Oberflächenrauhigkeit aufweisen, um eine möglichst einheitliche Beschickung der Reaktionsrohre mit Katalysatorfestbett zu gewährleisten.

Es ist aus dem Stand der Technik auch bekannt, dass heterogen katalysierte partielle Gasphasenoxidationen in den mit einem Katalysatorfestbett beschickten Reaktionsrohre eines Rohrbündelreaktors über vergleichsweise lange Zeiträume (bis zu mehreren Jahren) durchgeführt werden kann, ohne dass das Katalysatorfestbett in den Reaktionsrohren erneuert (frisch beschickt) werden muss (vgl. z. B. DE-A 103 50 822, DE-A 10 2004 025 445, EP-A 1 734 030 und den in diesen Schriften gewürdigten Stand der Technik). Ist das Katalysatorfestbett schließlich erschöpft, wird der Betrieb des Rohrbündelreaktors unterbrochen und das verbrauchte Katalysatorfestbett durch ein neues Katalysatorfestbett ersetzt (die Reaktionsrohre werden mit einem neuen Katalysatorfestbett wiederbeschickt). Anschließend wird der Rohrbündelreaktor wieder in Betrieb genommen und in dem neuen Katalysatorfestbett die gleiche oder eine andere heterogen katalysierte partielle Gasphasenoxidation einer organischen Verbindung durchgeführt.

Die US-A 4,701,101 empfiehlt nun im gegebenen Zusammenhang die Reaktionsrohre eines Rohrbündelreaktors, in welchem über längere Betriebszeiten eine heterogen katalysierte partielle Gasphasenoxidation durchgeführt worden ist, nach deren Beendigung sowie Entnahme des Katalysatorfestbetts in deren Innerem durch Sandstrahlen zu reinigen "to remove any vestiges of rust or of carbonaceous deposits on the walls of the tubes" (vgl. Übergang von Spalte 1 zu Spalte 2 in der US-A 4,701,101), bevor die Reaktionsrohre des Rohrbündelreaktors mit einem frischen Katalysatorfestbett wieder beschickt werden. Nachteilig an dieser Verfahrensweise ist jedoch, dass sie vergleichsweise aufwendig (es werden erhebliche Sandmengen benötigt, die nachfolgend entsorgt werden müssen) sowie vergleichsweise abrasiv wirkend ist (d. h., der Innendurchmesser der Reaktionsrohre wird bei mehrfacher Anwendung der Verfahrensweise zunehmend (über die verschiedenen Reaktionsrohre und deren Länge jedoch häufig uneinheitlich) aufgeweitet und verliert dadurch seine Abgestimmtheit mit der gewählten der verwendeten Katalysatorformkörper).
Ferner handelt es sich bei der Methode des Sandstrahlens zwar um eine sehr effektive Methode, die insbesonders hartnäckig auf der Innenwand der Reaktionsrohre zu entfernenden Belag zu beseitigen vermag, doch ist eine über die gesamte Reaktionsrohrlänge gleich bleibend effiziente Anwendung nur vergleichsweise schwierig zu erzielen, weshalb durch häufiges Sandstrahlen die Konstanz des Reaktionsrohrinnendurchmessers entlang seiner Länge verloren gehen und bei suboptimaler Anwendung der Verfahrensweise um den Mittelteil der Reaktionsrohrlänge (sie kann mehrere Meter betragen) herum leicht unerwünschter Belag verbleiben kann (ein solcher beeinträchtigt u. a. auch den Übergang der Reaktionswärme aus dem Reaktionsrohrinneren zum Wärmeaustauschmittel).
Als Alternative zur US-A 4,701,101 empfiehlt die JP-A 2006-159197 die Reaktionsrohre eines Rohrbündelreaktors, in welchem über längere Betriebszeiten eine heterogen katalysierte partielle Gasphasenoxidation durchgeführt worden ist, nach deren Beendigung sowie Entnahme des Katalysatorfestbetts in deren Innerem mit einer Flüssigkeit (vorzugsweise Wasser) zu waschen und nachfolgend zu trocknen, bevor die Reaktionsrohre des Rohrbündelreaktors mit einem frischen Katalysatorfestbett wieder beschickt werden. Nachteilig an dieser Verfahrensweise ist, dass sie nur bei solchen Wandbelägen auf der Innenseite der Reaktionsrohre greift, die in der verwendeten Waschflüssigkeit löslich sind. Nachteilig an dieser Verfahrensweise ist außerdem, dass die dabei resultierenden Suspensionen oder Lösungen in der Regel korrosiv sind und unter anderem zur Bildung von Flugrost und korrodierten Oberflächen (die u.a. auch deshalb nachteilig sind, weil sie die vollständige Oxidation zu katalysieren vermögen) führen.

Desweiteren ist diese Verfahrensweise insofern nicht unkritisch, als im wesentlichen alle Festbettkatalysatoren gegenüber Kontakt mit flüssigen Phasen empfindlich sind und ihre Wirksamkeit bei einem solchen Kontakt in der Regel wenigstens teilweise verlieren. D. h., wird die abschließende Trocknung der gewaschenen Reaktionsrohre (deren Gesamtzahl erstreckt sich in der Regel auf einige tausend) nicht wirklich quantitativ durchgeführt, so kann daraus eine Schädigung der neuen Katalysatorfestbettbeschickung resultieren (üblicherweise füllen sich die Poren der Aktivmassenoberfläche mit flüssiger Phase und entleeren sich von selbiger in der Regel nachträglich nur schwer, woraus eine Belegung der aktiven Zentren der Katalysatoroberfläche resultiert, was die Katalysatorperformance mindert).

Die Aufgabe der vorliegenden Erfindung bestand daher darin, eine gegenüber den beschriebenen Verfahrensweisen des Standes der Technik verbesserte Verfahrensweise zur Verfügung zu stellen, die deren Nachteile allenfalls nur noch in verminderter Form aufweist.

Demgemäss wurde ein Verfahren der Wiederbeschickung der Reaktionsrohre eines Rohrbündelreaktors mit einem neuen Katalysatorfestbett zum Zweck der Durchführung einer neuen heterogen katalysierten partiellen Gasphasenoxidation einer organischen Ausgangsverbindung in dem neuen Katalysatorfestbett, bei dem vor der Wiederbeschickung der Reaktionsrohre des Rohrbündelreaktors mit dem neuen Katalysatorfestbett in den selben Reaktionsrohren eine heterogen katalysierte partielle Vorgängergasphasenoxidation einer organischen Verbindung in einem in den Reaktionsrohren befindlichen Vorgängerkatalysatorfestbett, das wenigstens Katalysatorformkörper umfasste, deren Aktivmasse ein das Element Mo in oxidiertem Zustand enthaltendes Multielementoxid ist, unter Erzeugung eines Wasserdampf enthaltenden Produktgasgemischs durchgeführt worden und das Vorgängerkatalysatorfestbett nach Beendigung dieser Partialoxidation aus den Reaktionsrohren entnommen worden ist, gefunden, das dadurch gekennzeichnet ist, dass zwischen der Entnahme des Vorgängerkatalysatorfestbetts aus den Reaktionsrohren und der Wiederbeschickung dieser Reaktionsrohre mit dem neuen Katalysatorfestbett, wenigstens bei einem Teil der Reaktionsrohre ein auf deren Innenwand abgeschiedener, Molybdänoxid und/oder Molybdänoxidhydrat enthaltender, Feststoffbelag (Feststofffilm) mit Hilfe einer Bürste wenigstens teilweise weggebürstet wird.

Erfindungsgemäß bevorzugt erfolgt das Bürsten der Reaktionsrohre trocken, d. h., in Abwesenheit von flüssiger Phase.

Hintergrund der erfindungsgemäßen Verfahrensweise ist die Beobachtung, dass bei der Durchführung von heterogen katalysierten partiellen Gasphasenoxidationen in einem Katalysatorfestbett, das Katalysatorformkörper umfasst, deren Aktivmasse ein das Element Mo in oxidiertem Zustand enthaltendes Multielementoxid ist, und bei denen das Reaktionsgasgemisch (und damit auch das Produktgasgemisch) Wasserdampf enthält (von Anfang an als inertes Verdünnungsgas eingesetzt und/oder als Partialoxidationsnebenprodukt erst gebildet), im Verlauf des Langzeitbetriebs der heterogen katalysierten partiellen Gasphasenoxidation sukzessive Molybdänoxid (insbesondere Molybdäntrioxid und/oder dessen Hydrat) und/oder Hydrate von Molybdänoxiden (Molybdänoxidhydrat) aus der katalytischen Aktivmasse entweichen (z. B. durch Subblimation, die durch das Beisein von Wasserdampf gefördert wird) und sich auf der Innenwand des Reaktionsrohres als dünner Belag bzw. Film (mit einer Dicke von z. B. bis zu 0,5 mm) wieder niederschlagen. Diese Filme weisen aber eine nur vergleichsweise beschränkte Haftungsfähigkeit auf der Reaktionsrohrinnenoberfläche auf und können daher in voll befriedigender Weise durch konventionelles Bürsten wenigstens teilweise oder vollständig entfernt (weggebürstet) werden.

Weiterhin schützt der im wesentlichen aus Molybdänoxid und/oder Molybdänoxidhydrat (der letztere Begriff soll auch Molybdänhydroxide subsummieren) bestehende Film auf der Innenseite der Reaktionsrohre die selbigen vor ausgeprägter Rostbildung, weshalb in den erfindungsgemäßen Fällen die vergleichsweise drastische Maßnahme des Sandstrahlens zur Entfernung von im Verlauf der Partialoxidation gebildeten nennenswerten Rostmengen entfallen kann.

Vorteilhaft ist in diesem Zusammenhang auch, dass die Abscheidung von Molybdänoxid und/oder Molybdänoxidhydrat innerhalb der mit Katalysatorfestbett beschickten Reaktionsrohre dort besonders ausgeprägt ist, wo normalerweise die unerwünschte Bildung von Kohlenstoffablagerungen besonders problematisch ist. Dies ist üblicherweise in einer limitierten Zone in Strömungsrichtung hinter dem sogenannten Heißpunkt der Katalysatorfestbettbeschickung der Fall. Da heterogen katalysierte partielle Gasphasenoxidationen normalerweise ausgeprägt exotherme Reaktionen sind, ist die Temperatur des Reaktionsgasgemischs beim reaktiven Durchgang durch das Katalysatorfestbett von der Temperatur des das Katalysatorfestbett außerhalb der Kontaktrohre umströmenden fluiden Wärmeaustauschmittels normalerweise verschieden. Sie liegt üblicherweise oberhalb der Eintrittstemperatur des Wärmeaustauschmittels T_{w}^{ein} in die entsprechende Reaktionszone (Temperaturzone) und durchläuft längs einer Reaktionszone ein absolutes Maximum (Heißpunktmaximum) oder fällt von einem absoluten Maximalwert (gegebenenfalls über weitere relative Maxima) ausgehend ab. Dort wo vergleichsweise erhöhte Temperaturen des Reaktionsgasgemischs vorliegen ist nun aber nicht nur die Neigung zu thermischer Zersetzung der organischen Bestandteile des Reaktionsgasgemischs unter Ausbildung von sich in kälteren Regionen auf der Reaktionsrohrinnenfläche wieder abscheidenden, an Kohlenstoff vergleichsweise reichen, Bestandteilen erhöht, sondern auch die Übertrittsrate von Molybdänoxid und/oder Molybdänoxidhydrat in die Gasphase ist in diesen Regionen erhöht, weshalb sich im erfindungsgemäßen Fall die kohleartigen Abscheidungen im Unterschied zu jenen der US-A 4,701,101, wenn überhaupt in der Regel dort ausbilden, wo sich bereits ein Film aus Molybdän und/oder Molybdänoxidhydrat abgeschieden hat, weshalb es in den erfindungsgemäßen Fällen auch zur Beseitigung solcher kohleartigen Abscheidungen auf den Reaktionsrohrinnenwänden der vergleichsweise drastischen Entfernungsmethode des Sandstrahlens nicht bedarf. Vielmehr würden erfindungsgemäß solche kohleartigen Abscheidungen nicht unmittelbar auf der Reaktionsrohrinnenwand sondern auf den auf selbiger bereits abgeschiedenen Molybdänoxid- und/oder Molybdänoxidhydratfilmen abgeschieden. Sie vermögen daher nicht unmittelbar auf der Reaktionsrohrinnenwand festzubacken, sondern allenfalls auf den vergleichsweise mäßig auf der Reaktionsrohrinnenwand haftenden Filmen aus im wesentlichen Molybdänoxid und/oder Molybdänoxidhydrat, weshalb ihre Entfernung im erfindungsgemäßen Fall gleichfalls durch Bürsten erfolgen kann. Dies um so mehr, als man im Langzeitbetrieb einer erfindungsgemäßen heterogen katalysierten partiellen Gasphasenoxidation der über die Betriebszeit sich einstellenden Minderung der Aktivität des Katalysatorfestbetts in der Regel dadurch entgegenwirkt, dass T_{w}^{ein} des wenigstens einen Wärmeaustauschmittels sukzessive erhöht wird. Mit dieser Erhöhung geht normalerweise auch eine Erhöhung der erwähnten Heißpunkttemperaturen einher, die dann üblicherweise erst ab Überschreiten einer Grenztemperatur nennenswerte thermische Zersetzung bedingen. D. h., zu einem Zeitpunkt, wo die Ausbildung eines schützenden Molybdänoxid und/oder Molybdänoxidhydrat enthaltenden Films auf der Reaktionsrohrinnenfläche bereits erfolgt ist.

Unter einer vollständigen Oxidation einer organischen Verbindung mit molekularem Sauerstoff wird hier verstanden, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden in dieser Schrift als Partialoxidationen einer organischen Verbindung zusammengefasst.

Im besonderen sollen in dieser Schrift unter Partialoxidationen solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält als vor Durchführung der Partialoxidation.

Als ein sich unter den Bedingungen der heterogen katalysierten Gasphasenpartialoxidation im wesentlichen inert verhaltendes Verdünnungsgas werden in dieser Schrift solche Verdünnungsgase verstanden, deren Bestandteile im Reaktionsgasgemisch befindlich unter den Bedingungen der heterogen katalysierten partiellen Gasphasenoxidation - jeder Bestandteil für sich betrachtet - zu mehr als 95 mol-%, vorzugsweise zu mehr als 99 mol-% unverändert erhalten bleiben. Ihnen kommt die Aufgabe zu, einerseits einen Teil der Reaktionswärme aufzunehmen und als Bestandteil des Produktgasgemischs aus dem Rohrbündelreaktor herauszuführen und andererseits zu gewährleisten, dass sich das Reaktionsgasgemisch in der Regel außerhalb des explosionsfähigen Bereichs befindet. Für erfindungsgemäß relevante heterogen katalysierte partielle Gasphasenoxidationen geeignete inerte Verdünnungsgase sind z. B. N₂, CO₂, Wasserdampf, Edelgase und vielfach auch gesättigte Kohlenwasserstoffe (z. B. bei einer Partialoxidation von ungesättigten organischen Verbindungen) oder Mischungen aus allen oder Teilmengen der vorgenannten möglichen inerten Verdünnungsgase.

Unter Multielementoxiden (häufig Multimetalloxiden), die das Element Mo in oxidiertem Zustand enthalten, sollen in dieser Schrift Multielementoxide verstanden werden, in denen das Mo eine positive Oxidationszahl aufweist. Unter der Oxidationszahl des Mo wird dabei diejenige Ladungszahl verstanden, die das Mo hat, wenn man die bindenden Elektronen in dem betreffenden (in dem jeweiligen) Multielementoxid dem jeweils elektronegativeren Bindungspartner zuordnet (vgl. Grundlagen der allgemeinen und anorganischen Chemie, Verlag Sauerländer, Aarau, 1973, Seiten 73/74).
Besonders häufig weist das Mo in erfindungsgemäß relevanten Multielementoxiden die Oxidationszahl + VI auf.

Die Umsetzung der im Reaktionsgasgemisch einer heterogen katalysierten partiellen Gasphasenoxidation enthaltenen Reaktanden (O₂ und die organische Verbindung) erfolgt beim Hindurchführen des Reaktionsgasgemischs durch das in den Reaktionsrohren befindliche Katalysatorfestbett während der Verweilzeit der Reaktanden an der Katalysatoroberfläche.

Die Reaktionstemperatur in den Reaktionsrohren wird u. a. durch das wenigstens eine im Reaktionsrohrumgebungsraum geführte Wärmeaustauschmittel kontrolliert.

Die Reaktionsrohre im Rohrbündelreaktor sind, wie bereits erwähnt, in der Regel aus ferritischem Stahl oder aus Edelstahl gefertigt und weisen häufig eine Wanddicke von einigen mm, z. B. 1 bis 3 mm auf. Ihr Innendurchmesser beträgt meist einige cm, z. B. 10 bis 50 mm, häufig 20 bis 30 mm. Die Rohrlänge erstreckt sich im Normalfall auf wenige Meter (typisch ist eine Reaktionsrohrlänge im Bereich von 1 bis 10 m, häufig 2 bis 8 oder bis 6 m, vielfach 2 bis 4 m).

Anwendungstechnisch zweckmäßig beläuft sich die im Rohrbündelreaktor untergebrachte Anzahl an Reaktionsrohren (Arbeitsrohren) auf wenigstens 1000, häufig wenigstens 3000 oder 5000 und vielfach auf wenigstens 10000. Häufig beträgt die Anzahl der im Rohrbündelreaktor untergebrachten Kontaktrohre 15000 bis 30000 oder 40000 bis 50000. Rohrbündelreaktoren mit einer oberhalb von 50000 liegenden Anzahl von Kontaktrohren bilden eher die Ausnahme. Innerhalb des Reaktionsrohrumgebungsraums sind die Reaktionsrohre im Normalfall im wesentlichen homogen verteilt angeordnet, wobei die Verteilung zweckmäßig so gewählt wird, dass der Abstand der zentrischen Innenachsen von zueinander nächstliegenden Reaktionsrohren (die sogenannte Reaktionsrohrteilung) 25 bis 55 mm, häufig 35 bis 55 mm beträgt. Arbeitsrohre werden üblicherweise von Thermorohren unterschieden, wie sie z. B. die EP-A 873783 beschreibt. Während die Arbeitsrohre diejenigen Reaktionsrohre sind, in denen die heterogen katalysierte partielle Gasphasenoxidation im eigentlichen Sinn durchgeführt wird, dienen Thermorohre in erster Linie dem Zweck, die Reaktionstemperatur in den Reaktionsrohren zu verfolgen und zu steuern. Zu diesem Zweck enthalten die Thermorohre normalerweise zusätzlich zum Katalysatorfestbett eine lediglich mit einem Temperaturmessfühler beschickte, im Thermorohr längs desselben zentriert geführte Thermohülse. Im Regelfall ist die Anzahl der Thermorohre in einem Rohrbündelreaktor sehr viel kleiner als die Anzahl der Arbeitsrohre. Normalerweise beträgt die Anzahl der Thermorohre ≤ 20.

Erfindungsgemäß vorteilhaft wird beim erfindungsgemäßen Verfahren wenigstens bei 10 % aller Reaktionsrohre, vorzugsweise bei wenigstens 20 % oder bei wenigstens 30 % aller Reaktionsrohre, besonders vorteilhaft bei wenigstens 40 % oder bei wenigstens 50 % aller Reaktionsrohre, ganz besonders vorteilhaft bei wenigstens 60 % oder bei wenigstens 70 % aller Reaktionsrohre und am besten bei wenigstens 80 % oder bei wenigstens 90 % bzw. bei 100 % aller Reaktionsrohre zwischen der Entnahme des Vorgängerkatalysatorfestbetts aus den Reaktionsrohren und der Wiederbeschickung dieser Reaktionsrohre mit dem neuen Katalysatorfestbett, ein auf deren Innenwand abgeschiedener, Molybdänoxid und/oder Molybdänoxidhydrat enthaltender Feststoffbelag (Feststofffilm) mit Hilfe einer Bürste wenigstens teilweise weggebürstet. Erfindungsgemäß bevorzugt wird der relevante Feststoffbelag beim erfindungsgemäßen Bürsten zu wenigstens 25 % seines Gewichtes, besonders bevorzugt zu wenigstens 50 % seines Gewichtes oder zu wenigstens 75 % seines Gewichtes und ganz besonders bevorzugt zu wenigstens 90 Gew.-% oder zu wenigstens 95 % seines Gewichtes bzw. vollständig aus dem jeweils gebürsteten Reaktionsrohr entfernt.
Unter einer Bürste soll in dieser Schrift jedweder Formkörper verstanden werden, der durch ein Reaktionsrohr bewegt werden kann und dabei den relevanten Belag auf der Reaktionsrohrinnenwand abträgt (abreibt). In der Regel werden derartige Formkörper eine nicht glatte Oberfläche aufweisen. Anwendungstechnisch zweckmäßig sind sie so beschaffen, dass sie die Reaktionsrohrinnenwand nicht nennenswert beschädigen, wenn sie durch ein Reaktionsrohr bewegt werden. Die Längstausdehnung derartiger Formkörper (die längste direkte geradlinige Verbindungslinie zweier auf der Formkörperoberfläche befindlicher Punkte) beträgt normalerweise wenigstens 50 % des Innendurchmessers des erfindungsgemäß zu bürstenden Reaktionsrohres, in den meisten Fällen sogar wenigsten 75 %, bzw. wenigstens 100 %, oder wenigstens 125 % dieses Innendurchmessers. Im einfachsten Fall kann es sich z. B. um einen Molch (z. B. in Form eines kubischen Schaumstoffwürfels) mit rauer Oberfläche handeln, der z. B. die Innenwand des relevanten Reaktionsrohres mit seiner rauhen Oberfläche gerade berührt und z. B. unter Anwendung von Druckluft z. B. entgegengesetzt zur Schwerkraft durch das zu reinigende Reaktionsrohr getrieben werden kann.

Erfindungsgemäß bevorzugt kommen als solche Formkörper in erster Linie jedoch konventionelle Bürsten in Betracht, die normalerweise aus einem Grundkörper (z. B. aus Holz, Metall und/oder Kunststoff) und einem Bürstenbesatz (den Borsten) aufgebaut sind. Als Bürstenbesatz kommen für das erfindungsgemäße Verfahren sowohl Mineralfasern (z. B. Glasfasern), Naturfasern (z. B. tierische Borsten), synthetische Fasern (z. B. Nylon), synthetische Fasern mit Schleifmittelbeladung (z. B. Thonyl) sowie glatte oder gewellte Metalldrähte (z. B. ferritischer Stahl, Edelstahl, Messing, Bronze, verzinkter Stahl) in Betracht.

Als für ein erfindungsgemäßes Verfahren grundsätzlich verwendbare Bürsten kommen Walzenbürsten, Rundbürsten, Pipelinebürsten, Kegelbürsten, zylindrische Rohrbürsten, konische Rohrbürsten, rechteckige Rohrbürsten sowie Spiralbürsten in Betracht (wobei letztere erfindungsgemäß besonders bevorzugt sind).

Bei Bürsten mit gezopftem Besatz ist der Grundkörper normalerweise eine Blechronde. In die am Rand angeordneten Löcher werden z. B. Drahtbündel eingesteckt und dann verdrillt. Ihre Bauform ist in der Regel rund.

Bei gestanzten Bürsten werden Besatzbündel in die Löcher eines Grundkörpers eingeführt und verankert. Verankerungsmöglichkeiten sind Drahtschlingen oder spezielle Anker aus Blech.

Bei den Ringlochbürsten, bei denen es sich grundsätzlich um runde Bürsten handelt, wird der Besatz zwischen einem Rohr und einem Ring eingepresst. Ihre Bauform ist wie bereits gesagt immer rund (Höhe (Länge) ≤ Außendurchmesser). In Segmenten zusammengestellt sind sie auch als Walzen (Höhe (Länge) > Außendurchmesser) ausführbar.

Bei den Steifenbürsten ist die Grundkonstruktion meistens ein gebogener Blechstreifen, in dessen Falz der Besatz durch einen Haltedraht fixiert wird. Grundsätzlich kann die Grundkonstruktion dieser Bürstenart auch aus Kunststoffen hergestellt sein. Ihre Bauformen können z. B. gerade, wendelförmig, walzenförmig (um eine Welle gewickelt) oder frei geformt sein.

In erfindungsgemäß günstigen Fällen wird der Bürstenkörper (der Grundkörper) nicht aufgeschnitten, sondern lediglich sehr fein aufgebohrt. Durch diese Bohrungen werden Drähte gezogen, um welche die Borstenbündel einzeln geführt werden. Anschließend werden Bohrungen durch kleine Stopfen an der Stirnseite verplombt. Dieses aufwendige Verfahren sorgt für eine sehr hohe Langlebigkeit der Bürste.

Über den Bürstendurchmesser kann u. a. die Umfangsgeschwindigkeit bzw. die Axialgeschwindigkeit und damit die Leistung der Bürste mitgesteuert werden. Der Durchmesser der Bürste und die Länge des jeweiligen Besatzes sind bestimmende Faktoren für das Ergebnis des erfindungsgemäßen Reaktionsrohrbearbeitungsprozesses. Aus der Kombination von kleinerem Bürstenkörper-Durchmesser und höherer Besatzlänge resultieren vergleichsweise flexible ("weiche") Bürsten, deren Verwendung eine besonders schonende Oberflächenbearbeitung gewährleistet. Ein größerer Bürstenkörper-Durchmesser in Kombination mit kurzen Besatzlängen (Borstenlängen) des Besatzmaterials ergibt aggressivere ("härtere") Bürsten, die z. B. im Fall von stärkerer erfindungsgemäß relevanter Belagsbildung eingesetzt werden können.

Erfindungsgemäß vorteilhaft wird man das erfindungsgemäße Verfahren so durchführen, dass man mit einer "härteren" Bürste beginnt und das Verfahren mit einer "weicheren" Bürste beendet. Diese Vorgehensweise gewährleistet auf besonders einfache Art und Weise, dass die Oberfläche der Reaktionsrohrinnenseite beim erfindungsgemäßen Bürsten nicht verkratzt und/oder bei mehrfacher Anwendung nicht abrasiv abgetragen wird.

Selbst das Anforderungsprofil für die Rauheit der Reaktionsrohrinnenfläche gemäß der Lehre der JP-A 2006-142288 lässt sich bei dieser Vorgehensweise auch über einen vieljährigen Betrieb des Rohrbündelreaktors auf erfindungsgemäß einfache Art und Weise erfüllen.

Die Besatzdichte einer Bürste ist die Anzahl der Drahtspitzen pro Oberflächeneinheit des Grundkörpers der Bürste. Hohe Besatzdichten gewährleisten üblicherweise gute Ergebnisse bei Anwendung des erfindungsgemäßen Verfahrens und eine erhöhte Standzeit der verwendeten Bürste. Ein spiralförmiger Besatz (insbesondere im Fall eines kreiszylindrischen Grundkörpers (d. h., z. B. im Fall einer Rundbürste oder Walzenbürste bzw. Rohrbürste)) ist für das erfindungsgemäße Verfahren, wie bereits gesagt, besonders bevorzugt. Auf eine biegsame Rotationswelle (in der Regel weisen erfindungsgemäß geeignete Bürsten einen einseitigen oder beidseitigen Wellenansatz auf) aufgebracht zieht sich die Bürste quasi von alleine in das zu bürstende Reaktionsrohr hinein (im Fall einer Übereinstimmung von Drehsinn der Welle und Drehsinn der Spirale). Es ist auf diese Weise besonders einfach, die Reinigungsbürste im zu reinigenden Reaktionsrohr vorwärtszutreiben. Dabei sind Anstiegswinkel der Spirale von 5 bis 50 °, vorzugsweise von 10 bis 40 ° bzw. 15 bis 25 ° oder 25 bis 35 ° besonders vorteilhaft.
Anstelle die Bürste an einer rotierenden Welle ins Reaktionsrohr zu führen, kann die Bürste in einer einfacheren Ausführungsform des erfindungsgemäßen Verfahrens auch an einer Stange oder an einer Feder befestigt im Rohr hin- und herbewegt werden. Anstelle einer einfachen Besatzspirale kann eine erfindungsgemäß geeignete Rund(oder Walz-, oder Rohr-)bürste auch mit einer zwei- oder mehrfachen Besatzspirale ausgeführt sein.
Die Borstendicke kann bei erfindungsgemäß geeigneten Bürsten z. B. 0,08 bis 1,20 mm betragen.
Erfindungsgemäß bevorzugt ist eine Borstendicke (insbesondere im Fall von Metalldrähten), die im Bereich von 0,1 bis 0,5 mm liegt.

Grundsätzlich ist es beim erfindungsgemäßen Verfahren anwendungstechnisch vorteilhaft, wenn der Bürstendurchmesser nicht größer als die lichte Weite des zu reinigende Reaktionsrohres ist.
Anwendungstechnisch zweckmäßig liegt der Bürstendurchmesser 0,1 bis 2 mm, häufig 0,5 bis 1,5 mm unterhalb der lichten Weite des zu reinigenden Reaktionsrohres (die lichte Weite bezieht sich dabei stets auf das von Belägen freie Reaktionsrohr).

Erfindungsgemäß besonders vorteilhaft sind kreiszylindrische Rohrbürsten mit spiralförmig ausgeführtem Besatz. Die Zylinderlänge beträgt dabei vorteilhaft 4 bis 25 cm, besonders vorteilhaft 6 bis 15 cm. Als Besatzmaterial kann auch das selbe Material verwendet werden, aus welchem die Reaktionsrohre selbst gefertigt sind. Vorzugsweise ist der Drahtbesatz dabei glatt und nicht gewellt. Die Besatzlänge beträgt in der Regel nicht weniger als 20 % des Reaktionsrohrinnenradius und üblicherweise nicht mehr als 80 % des Reaktionsrohrinnenradius. Ein erfindungsgemäß ganz generell günstiges Besatzmaterial sind z. B. die DIN-Werkstoffe 1.4310 und 1.4401. Selbstverständlich können für diesen Zweck aber auch die DIN-Werkstoffe 1.4301 und 1.4571 verwendet werden.

Wird die Bürste im Reaktionsrohr über eine Welle angetrieben, liegen erfindungsgemäß typische Umfangsgeschwindigkeiten im Fall von glattem Besatz (insbesondere im Fall von Metalldraht) bei 25 bis 35 m/sec und im Fall von gewelltem Besatz bei 15 bis 25 m/sec.

Der beim erfindungsgemäßen Verfahren erzeugte Abrieb des Reaktionsrohrinnenbelags kann in der einfachsten Ausführungsform am unteren Ende des Reaktionsrohres aufgefangen werden (er fällt normalerweise durch die Maschen des Trägerrostes für die Katalysatorformkörper). In vorteilhafter Weise wird der Trägerrost vorab der erfindungsgemäßen Reaktionsrohrreinigung entfernt. In einer anwendungstechnisch bevorzugten Ausgestaltung der erfindungsgemäßen Verfahrensweise wird der durch das Bürsten erzeugte Abrieb unmittelbar bei seiner Entstehung aus dem jeweiligen Reaktionsrohr abgesaugt. Eine derartige Vorgehensweise ist unter sicherheitstechnischen Gesichtspunkten zu bevorzugen. Dabei kann die wenigstens eine Saugöffnung im Grundkörper der Bürste und/oder in einem parallel zur Bürste ins Reaktionsrohr eingeführten separaten elastischen Saugschlauch angebracht sein. Im Fall einer solchen saugenden Ausführungsform des erfindungsgemäßen Verfahrens ist die Reaktionsrohröffnung im oberen Rohrboden des Rohrbündelreaktors bei Durchführung des Verfahrens normalerweise abgedichtet (eingedichtet) verschlossen und die die Bürste antreibende Welle und/oder der Saugschlauch werden in diesem Verschluß gleichfalls eingedichtet geführt.

Das erfindungsgemäße Verfahren ist ein vergleichsweise anspruchvolles und immer noch aufwendiges Verfahren (im Mittel verbraucht die erfindungsgemäße Reinigung von 20 bis 40 Reaktionsrohren eine Bürste; die erfindungsgemäße Reinigung von 22000 Reaktionsrohren beansprucht etwa 200 Mannstunden). Nichtsdestotrotz ist es den Verfahren des Standes der Technik aber nicht zuletzt dadurch überlegen, dass es das Material der Reaktionsrohrwand vergleichsweise erhaltend und dennoch mit hoher Reinigungseffizienz durchgeführt werden kann. Eine endoskopische Inspektion der Reaktionsrohre gibt dabei über die Reinigungswirkung Auskunft. Darüber hinaus ist die erfindungsgemäße Verfahrensweise ubiquitär anwendbar.

Erfindungsgemäß ganz besonders vorteilhaft ist der Rohrbündelreaktor aus ferritischem Stahl vom DIN Typ 1.0425 gefertigt. Für die Reaktorböden, Reaktionsrohre und Reaktorhauben wird häufig auch Stahl der DIN-Werkstoffnummer 1.0481 oder 1.0315 und für den Reaktormantel vielfach Stahl der DIN-Werkstoffnummer 1.0345 verwendet. Diejenige Reaktorhaube, über die das Reaktionsgaseingangsgemisch zuströmt, ist dabei jedoch anwendungstechnisch vorteilhaft mit austenitischem Stahl (vorzugsweise vom DI N-Typ 1.4541 oder vom DI N Typ 1.4571) plattiert. Typische Plattierungsstärken liegen bei etwa 3 mm.

Insbesondere bei Rohrbündelreaktoren mit größerem Querschnitt ihrer Rohrböden ist es anwendungstechnisch zweckmäßig, im Zentrum des Rohrbündelreaktors einen unberohrten Bereich zu belassen, und statt dessen in diesem Bereich den oberen Rohrboden abzustützen. Die Aussage, dass die Reaktionsrohre in die Durchgangsöffnungen im oberen bzw. unteren Rohrboden eingedichtet sind, bringt zum Ausdruck, dass zwischen der Reaktionsrohraußenwand und der Bohrungswand (bzw. der Wand der Durchgangsöffnung, oder auch Einhüllenden der Durchgangsöffnung) keine Durchtrittsmöglichkeit für das Wärmeaustauschmittel besteht. Eine solche Eindichtung kann z. B. wie in der DE 202006014116 U1 beschrieben erfolgen. In entsprechender Weise ist auch der Umfang des oberen bzw. unteren Rohrbodens so in den Reaktormantel des Rohrbündelreaktors eingearbeitet, dass zwischen beiden keine Durchgangsmöglichkeit für das Wärmeaustauschmittel besteht. Im oberen Rohrboden befindet sich im Fall der Verwendung eines flüssigen Wärmeaustauschmittels in der Regel jedoch eine Verbindung zur Wärmeaustauschmittelpumpe, die eine Entgasung des Reaktionsrohrumgebungsraums gewährleistet und sicherstellt, dass das flüssige Wärmeaustauschmittel den oberen Rohrboden benetzt (vgl. z. B. EP-A 987057). Im übrigen ist ein Einzonenrohrbündelreaktor vorzugsweise so wie in der DE-A 4431949 beschrieben gestaltet.
Die Temperatur T_{w}^{ein} des wenigstens einen (vorzugsweise flüssigen) Wärmeaustauschmittels liegt bei der heterogen katalysierten partiellen Vorgängergasphasenoxidation einer organischen Verbindung in typischer Weise im Bereich von 200 bis 500 °C, häufig im Bereich von 250 bis 400 °C und vielfach im Bereich von 250°C bis 310°C.

Der Wasserdampfgehalt des Reaktionsgaseingangsgemischs kann bei der heterogen katalysierten partiellen Vorgängergasphasenoxidation grundsätzlich 0 (verschwindend) sein.

In diesen Fällen ist es erfindungsgemäß wesentlich, dass H₂O als Nebenprodukt der heterogen katalysierten partiellen Vorgängergasphasenoxidation gebildet wird. Im übrigen eignet sich das erfindungsgemäße Verfahren insbesondere dann, wenn bereits der Wasserdampfgehalt des Reaktionsgaseingangsgemischs bei der Vorgängergasphasenpartialoxidation > 0 Vol.-% beträgt. Es ist insbesondere dann vorteilhaft, wenn dieser Wasserdampfgehalt des Reaktionsgaseingangsgemischs ≥ 0,1 bis 60 Vol.-%, oder ≥ 0,2 bis 50 Vol.-%, oder ≥ 0,3 bis 40 Vol.-%, oder ≥ 0,4 bis 30 Vol.-%, oder ≥ 0,5 bis 25 Vol.-%, oder ≥ 0,75 bis 20 Vol.-%, oder ≥ 1 bis 15 Vol.-%, oder ≥ 2 bis 10 Vol.-% beträgt. Wasserdampf ist aufgrund seiner spezifischen Wärmekapazität in der Regel ein hervorragendes inertes Verdünnungsgas für Vorgängergasphasenpartialoxidationen und wirkt sich vielfach förderlich auf die Katalysatoraktivität aus.

Der Arbeitsdruck kann bei einer heterogen katalysierten Vorgängergasphasenpartialoxidation sowohl unterhalb von Normaldruck (z. B. bis zu 0,5 bar, das Reaktionsgasgemisch wird durchgesaugt) als auch oberhalb von Normaldruck liegen. Typischerweise wird der vorgenannte Arbeitsdruck bei Werten von 1 bis 5 bar, häufig 1,5 bis 3,5 bar liegen. Normalerweise wird der Arbeitsdruck bei einer Vorgängergasphasenpartialoxidation 100 bar nicht überschreiten.

Als Quelle für den für eine Vorgängergasphasenpartialoxidation erforderlichen molekularen Sauerstoff im Reaktionsgaseingangsgemisch kommen sowohl Luft, reiner molekularer Sauerstoff, an molekularem Stickstoff entreicherte Luft oder sonstige Gemische aus Inertgas und molekularem Sauerstoff in Betracht.

Der Gehalt des Reaktionsgaseingangsgemischs an der heterogen katalysiert partiell zu oxidierenden organischen Verbindung kann bei einer Vorgängergasphasenpartialoxidation bis zu 50 Vol.-% oder mehr betragen. Häufig wird dieser Gehalt ≥ 2 bis 20 Vol.-%, oder ≥ 4 bis 12 Vol.-% betragen. Enthält das Reaktionsgaseingangsgemisch den molekularen Sauerstoff in einer bezogen auf die angestrebte Partialoxidation unterstöchiometrischen Menge, kann die im Reaktionsgaseintrittsgemisch enthaltene Überschussmenge an der partiell zu oxidierenden organischen Verbindung grundsätzlich als inertes Verdünnungsgas fungieren. Enthält das Reaktionsgaseingangsgemisch molekularen Sauerstoff in auf die Partialoxidation bezogen überstöchiometrischer Menge, wird selbige dabei anwendungstechnisch vorteilhaft häufig so gewählt, dass die Zusammensetzung des Reaktionsgaseingangsgemischs außerhalb des explosionsfähigen Zusammensetzungsbereichs liegt.

Aus Gründen einer möglichst langen Katalysatorstandzeit wird man den Anteil des molekularen Sauerstoff im Reaktionsgaseingangsgemisch einer Vorgängergasphasenpartialoxidation in der Regel zweckmäßig so wählen, dass das Produktgasgemisch der Vorgängergasphasenpartialoxidation noch überschüssigen molekularen Sauerstoff (z. B. bis zu 3 Vol.-%) enthält.

Der Volumenstrom des Temperiermediums (des wenigstens einen Wärmeaustauschmittels (vorzugsweise eines flüssigen)) im Reaktionsrohrumgebungsraum wird bei einer Vorgängergasphasenpartialoxidation üblicherweise so bemessen, dass der Temperaturanstieg (bedingt durch die Exothermie der Reaktion) des (vorzugsweise flüssigen) wenigstens einen Wärmeaustauschmittels von seiner Eintrittsstelle in den Reaktor bis zu seiner Austrittstelle aus dem Reaktor ≥ 0 bis 15 °C bzw. bis 10 °C, häufig ≥ 2 bis 8 °C, oft ≥ 3 bis 6 °C beträgt.

Die Belastung des Katalysatorfestbetts mit der partiell zu oxidierenden organischen Verbindung wird bei einer Vorgänger-Gasphasenpartialoxidation in der Regel ≥ 50 NI/l•h, meist ≥ 75 NI/l•h, vielfach ≥ 100 NI/l•h betragen. Meist wird diese Belastung jedoch bei ≤ 600 NI/l•h liegen.

Die Belastung des Katalysatorfestbetts mit Reaktionsgaseingangsgemisch wird bei einer Vorgängergasphasenpartialoxidation häufig ≥ 1500 NI/l•h, oder ≥ 2000 NI/l•h, oder ≥ 2500 NI/l•h, oder ≥ 3000 NI/l•h, oder ≥ 4000 NI/l•h betragen. In der Regel liegt vorgenannte Belastung bei Vorgängergasphasenpartialoxidationen jedoch bei Werten von ≤ 6000 NI/l•h, bzw. ≤ 5000 NI/l•h.

Der Umsatz der partiell zu oxidierenden organischen Verbindung wird bei einer Vorgängergasphasenpartialoxidation in typischer Weise ≥ 50 mol-%, häufig ≥ 70 mol-%, vielfach ≥ 80 mol-% und oft ≥ 90 mol-% betragen (bezogen auf den einmaligen Durchgang des Reaktionsgasgemischs durch das Katalysatorfestbett). Die Selektivität der Zielproduktbildung wird dabei in typischer Weise ≥70 mol-%, häufig ≥ 80 mol-% und vielfach ≥ 90 mol-% betragen.

Im übrigen werden die Rahmenbedingungen einer heterogen katalysierten Vorgängergasphasenpartialoxidation anwendungstechnisch zweckmäßig insgesamt normalerweise so gewählt, dass der Temperaturunterschied zwischen dem Heißpunktmaximum des Reaktionsgasgemischs in den einzelnen Reaktionszonen (Temperaturzonen) des Rohrbündelreaktors und der jeweiligen zugehörigen T_{w}^{ein} der Temperaturzone auch im Langzeitbetrieb in der Regel 80 °C nicht überschreitet. Meist beträgt dieser Temperaturunterschied auch im Langzeitbetrieb ≤ 70 °C, häufig liegt er bei 20 bis 70 °C bzw. bis 50 °C, vorzugsweise ist dieser Temperaturunterschied auch im Langzeitbetrieb gering.

Außerdem werden die vorgenannten Rahmenbedingungen üblicherweise so gewählt, dass die "peak-to-salt temperatur sensitivity (vgl. Definition in der EP-A 1106598) insbesondere auch im Langzeitbetrieb ≤ 9 °C, aber ≤ 7 °C, oder ≤ 5 °C, oder ≤ 3 °C beträgt.

In vielen Fällen ist bei einer erfindungsgemäßen heterogen katalysierten partiellen Vorgängergasphasenoxidation bei wenigstens 25 Gew.-%, oder bei wenigstens 50 Gew.-%, oder bei wenigstens 75 Gew.-% oder bei der Gesamtmenge der Katalysatorformkörper des in den Reaktionsrohren befindlichen Katalysatorfestbetts die Aktivmasse so beschaffen, dass sie ein das Element Mo in oxidiertem Zustand enthaltendes Multielementoxid ist.

Üblicherweise handelt es sich bei solchen Mo in oxidiertem Zustand enthaltenden Multielementoxiden um solche Multielementoxide, die neben Mo noch wenigstens eines der Elemente Bi, V, P und Fe in oxidiertem Zustand enthalten. Grundsätzlich meint der Begriff Multielementoxid, dass die katalytisch aktive Oxidmasse neben Sauerstoff und Mo noch wenigstens ein anderes Element (in der Regel mehr als ein anderes Element) enthält. Besonders häufig kommen als katalytisch aktive Oxidmassen für die Vorgängergasphasenpartialoxidation solche (insbesondere Mo-haltige) zur Anwendung, die mehr als ein metallisches, insbesondere übergangsmetallisches Element umfassen. In diesem Fall spricht man von Multimetalloxidmassen. Im Regelfall sind katalytisch aktive Multielementoxidmassen (insbesondere die Mo-enthaltenden) keine einfachen physikalischen Gemische von Oxiden ihrer elementaren Konstituenten, sondern heterogene Gemische von komplexen Polyverbindungen dieser Elemente.

Die katalytisch wirksame Aktivmasse der Katalysatoren des Katalysatorfestbetts einer Vorgängergasphasenpartialoxidation (insbesondere im Fall von Mo-haltigen Multielementoxidaktivmassen) werden normalerweise zu Formkörpern unterschiedlichster Geometrie (als sogenannte geometrische Katalysatorformkörper) geformt verwendet, um in den Reaktionsrohren des Rohrbündelreaktors das Katalysatorfestbett zu gestalten (die Reaktionsrohre mit dem Katalysatorfestbett zu beschicken). Beispielsweise kommen als solche geometrischen Formkörper Kugeln, Tabletten, Stränge, Ringe, Spiralen, Pyramiden, Zylinder, Prismen, Quader, Würfel etc. in Betracht.

Dabei kann der geometrische Formkörper im einfachsten Fall nur aus katalytisch aktiver Masse, die gegebenenfalls mit Formgebungshilfsmitteln und/oder inertem Material verdünnt sein kann, bestehen. Solche geometrischen Katalysatorformkörper werden üblicherweise als Vollkatalysatoren bezeichnet.

Im Fall von Vollkatalysatoren kann die Formgebung z. B. dadurch erfolgen, dass man die katalytisch aktive Pulvermasse (z. B. eine pulverförmige Multielementoxidaktivmasse) zur gewünschten Katalysatorgeometrie verdichtet (z. B. durch Tablettieren, Sintern, Extrudieren oder Strangpressen). Dabei können Formungshilfsmittel zugesetzt werden. Alternativ kann man eine pulverförmige Vorläufermasse zur gewünschten Katalysatorgeometrie verdichten und den resultierenden geometrischen Formkörper durch thermisches Behandeln (gegebenenfalls in molekularen Sauerstoff enthaltender Atmosphäre) in den katalytisch aktiven Multielementoxidformkörper überführen (vgl. z. B. US 2005/0263926).

Selbstverständlich kann die Formgebung auch so erfolgen, dass man einen geometrischen Formkörper aus katalytisch nicht aktivem Material (aus Inertmaterial) mit Aktivmasse beschichtet (nachstehend auch als "Trägerformkörper" oder kurz als "Trägerkörper" bezeichnet). Alternativ kann auch mit Vorläufermasse beschichtet werden und die Überführung in den aktiven Katalysator durch nachträgliche thermische Behandlung erfolgen (gegebenenfalls in molekularen Sauerstoff enthaltender Atmosphäre). Das Beschichten kann in einfachster Weise z. B. dadurch erfolgen, dass man die Oberfläche eines inerten Trägerkörpers mittels eines flüssigen Bindemittels befeuchtet und nachfolgend pulverförmige Aktivmasse oder pulverförmige Vorläufermasse an der befeuchteten Oberfläche anheftet. Die auf diese Wiese erhältlichen Katalysatoren werden als Schalenkatalysatoren bezeichnet.

Geeignete inerte Trägerkörper sind für viele heterogen katalysierte partielle Gasphasenoxidationen poröse oder unporöse Aluminiumoxide, Siliciumoxid, Thoriumdioxid, Zirkonoxid, Siliciumcarbid oder Silikate wie Magnesium- oder Aluminiumsilicat (z. B. Steatit des Typs C220 der Fa. CeramTec), aber auch Metalle wie z. B. Edelstahl oder Aluminium (vgl. z. B. US 2006/0205978).

Anstelle den inerten (inert heißt hier in der Regel, dass wenn das Reaktionsgasgemisch untern den Reaktionsbedingungen durch ein nur mit inerten Trägerkörpern beschicktes Kontaktrohr geführt wird, der Umsatz der Reaktanden ≤ 5 mol-%, meist ≤ 2 mol-% beträgt) Trägerkörper mit pulverförmiger Aktivmasse oder mit pulverförmiger Vorläufermasse zu beschichten, kann man den Trägerkörper in vielen Fällen auch mit einer Lösung der katalytisch aktiven Substanz oder mit einer Lösung einer Vorläufersubstanz tränken und nachfolgend das Lösungsmittel verflüchtigen und gegebenenfalls eine chemische Reduktion und/oder thermische Behandlung (gegebenenfalls in molekularen Sauerstoff enthaltender Atmosphäre) anschließen. Die auf dieser Weise resultierenden geometrischen Katalysatorformkörper werden üblicherweise als Träger- oder Tränkkatalysatoren bezeichnet.

Unter der Längstausdehnung L solcher geometrischer Katalysatorformkörper (wie ganz generell von geometrischen Formkörpern in dieser Schrift) wird die längste mögliche direkte Verbindungslinie zweier auf der Oberfläche des Katalysatorformkörpers befindlicher Punkte verstanden. Sie beträgt (auch bei geometrischen Inertformkörpern) meist 1 bis 20 mm, oft 2 bis 15 mm und vielfach 3 bzw. 4 bis 10 bzw. bis 8 oder bis 6 mm. Im Fall von Ringen liegt die Wanddicke darüber hinaus üblicherweise bei 0,5 bis 6 mm, häufig bei 1 bis 4 bzw. bis 3 oder bis 2 mm.

Nicht bei allen heterogen katalysierten partiellen Vorgängergasphasenoxidationen am in den Rohren von Rohrbündelreaktoren befindlichen Katalysatorfestbett besteht das Katalysatorfestbett aus einer längs des individuellen Kontaktrohres einheitlichen Schüttung aus einer einzigen Sorte geometrischer Katalysatorformkörper. Vielmehr kann das Katalysatorfestbett über die Gesamtlänge des Kontaktrohres auch aus einem homogenisierten Gemisch mehrerer (d. h., wenigstens zwei) voneinander unterscheidbarer Sorten Sⁱ von geometrischen Katalysatorformkörpern oder von geometrischen Katalysatorformkörpern und geometrischen Inertformkörpern bestehen (d. h., ein solches Gemisch kann aus wenigstens zwei voneinander unterscheidbaren Sorten von geometrischen Katalysatorformkörpern, oder aus einer einzigen Sorte von geometrischen Katalysatorformkörpern und aus einer einzigen Sorte von geometrischen Inertformkörpern, oder aus wenigstens zwei Sorten von voneinander unterscheidbaren geometrischen Katalysatorformkörpern und einer einzigen Sorte von geometrischen Inertformkörpern, oder aus wenigstens zwei Sorten von voneinander unterscheidbaren geometrischen Katalysatorformkörpern und wenigstens zwei Sorten von voneinander unterscheidbaren geometrischen Inertformkörpern bestehen). Mögliche Unterscheidungsmerkmale der voneinander verschiedenen Sorten Sⁱ sind die Art der Geometrie, die Art der Aktivmasse, die Art des Trägermaterials etc.. Als Materialien für die geometrischen Inertformkörper kommen die gleichen Materialien in Betracht, die bereits für die inerten geometrischen Trägerformkörper bei den Schalenkatalysatoren empfohlen wurden und in den Ablauf der Gasphasenpartialoxidation im wesentlichen nicht eingreifen. Grundsätzlich kommen alle inerten Trägerformkörper auch als geometrische Inertformkörper zur Verdünnung von geometrischen Katalysatorformkörpern in einem Katalysatorfestbett in Betracht. Durch eine solche Verdünnung kann die volumenspezifische Aktivität eines Katalysatorfestbetts auf den Bedarf der jeweiligen heterogen katalysierten partiellen Gasphasenoxidation spezifisch eingestellt werden.

Der Wortlaut "homogenisiertes Gemisch" meint dabei, dass Maßnahmen ergriffen worden sind, um die voneinander verschiedenen Sorten geometrischer Formkörper (bzw. die verschiedenen Längstausdehnungen innerhalb einer Sorte) miteinander homogen zu vermengen. Im Idealfall erreicht die homogene Vermengung entlang des gesamten Längsabschnitts den statistischen Durchschnitt und dies auch bezüglich der jeweiligen individuellen Sorte.

Vielfach besteht eine Kontaktrohrbeschickung (eine Kontaktrohrbefüllung) mit einem Katalysatorfestbett aber auch aus mehreren voneinander unterscheidbar übereinander (hintereinander) angebrachten Längsabschnitten (Katalysatorfestbett(längs)abschnitten, Katalysatorschüttungsabschnitten). Jeder einzelne Längsabschnitt kann dabei über seine Länge einheitlich so gestaltet werden, wie es für ein über seine gesamte Kontaktrohrlänge einheitlich beschicktes Kontaktrohr bereits ausgeführt wurde. Beim Übergang von einem in sich einheitlichen Schüttungsabschnitt zum nächsten in sich einheitlichen Schüttungsabschnitt ändert sich die Zusammenstellung (Zusammensetzung) der Schüttung abrupt. Es entstehen so längs eines individuellen Kontaktrohres Katalysatorfestbettschüttungen, die eine heterogene Struktur aufweisen. Man spricht auch von einer strukturierten Befüllung (bzw. Schüttung) der Kontaktrohre. Am Anfang (in Strömungsrichtung des das Kontaktrohr durchströmenden Reaktionsgases geblickt) und/oder am Ende des Kontaktrohres wird das Katalysatorfestbett häufig durch eine alleinige Schüttung aus geometrischen Inertformkörpern abgeschlossen.

Beispiele für solche strukturierten Befüllungen von Kontaktrohren sind unter anderem in den Schriften US 2006/0161019, EP-A 979 813, EP-A 090 744, EP-A 456 837, EP-A 1 106 598, US 5,198,581 und US 4,203,903 beschrieben.

In der Regel wird die Befüllung eines Kontaktrohres mit einem strukturierten Katalysatorfestbett so gestaltet, dass die volumenspezifische Aktivität des Katalysatorfestbetts in Strömungsrichtung des Katalysatorfestbetts zunimmt. Die volumenspezifische Aktivität eines in sich einheitlichen Längsabschnittes einer Katalysatorfestbettbeschickung eines Kontaktrohres ist dann erhöht, wenn bei durchgehender Beschickung des Kontaktrohres wie im entsprechenden Längsabschnitt des Kontaktrohres unter ansonsten identischen Reaktionsbedingungen (d. h., identische Zusammensetzung des Reaktionsgasgemischs, identische Belastung der Katalysatorfestbettbeschickung mit Reaktionsgasgemisch sowie identische Eintrittstemperatur des Wärmeträgers und identische Strömungsbedingungen des Wärmeträgers) ein erhöhter Eduktumsatz (bezogen auf den einmaligen Durchgang des Reaktionsgasgemischs durch das Kontaktrohr) resultiert.

Das in dieser Schrift Gesagte gilt insbesondere dann, wenn das Vorgängerkatalysatorfestbett wenigstens Katalysatorformkörper umfasst (oder zu wenigstens 25 %, bzw. zu wenigstens 50 %, oder zu wenigstens 75 %, bzw. zu 100 % seines Gewichtes nur aus solchen Katalysatorformkörpern besteht), deren Aktivmasse ein Multielementoxid ist, das das Element Mo in oxidiertem Zustand enthält, mit der Maßgabe, dass der molare stöchiometrische Koeffizient des Mo innerhalb der von Sauerstoff verschiedenen Elemente der Multielementoxidaktivmasse den höchsten Wert aufweist.

Alles in dieser Schrift Gesagte ist insbesondere dann zutreffend, wenn das Vorgängerkatalysatorfestbett wenigstens Katalysatorformkörper umfasst, deren Aktivmasse ein Multielementoxid der allgemeinen Formel I,

Mo₁₂BiₐFe_{b}X_{c}¹X_{d}²Xₑ³X_{f}⁴Oₙ (I),

mit
- X¹ =: Nickel und/oder Kobalt,
- X² =: Thallium, ein Akalimetall und /oder ein Erdalkalimetall,
- X³ =: Zink, Phosphor, Arsen, Bor, Antimon, Zinn, Cer, Blei, Vanadium, Chrom und/oder Wolfram,
- X⁴ =: Silizium, Aluminium, Titan und/oder Zirkonium,
- a =: 0,2 bis 5,
- b =: 0,01 bis 5,
- c =: 0 bis 10,
- d =: 0 bis 2,
- e =: 0 bis 8,
- f =: 0 bis 10, und
- n =: eine Zahl, die durch die Wertigkeit und Häufigkeit der von Sauerstoff verschie denen Elemente in I bestimmt wird,
ist.

Alles in dieser Schrift Gesagte ist insbesondere dann zutreffend, wenn das Vorgängerkatalysatorfestbett zu wenigstens 25 %, oder zu wenigstens 50 %, oder wenigstens 75 % seines Gewichtes, oder nur aus Katalysatorformkörpern besteht, deren Aktivmasse ein Multielementoxid I ist (wie stets in dieser Schrift werden bei der Berechnung der Gewichtsprozentanteile reine Inertschüttungen innerhalb des Katalysatorfestbetts nicht miteinbezogen).

Die Herstellung entsprechender (insbesondere ringförmiger) Vollkatalysatoren sowie Schalenkatalysatoren findet sich z. B. beschrieben in der US 2005/0263926, in der DE-A 10313209, in der WO 02/30569, in der WO 2005/030393, in Research Disclosure RD 2005/497012, in der DE-A 102007005606 sowie in der DE-A 102007004961 (und dem in diesen Schriften zitierten Stand der Technik).

In den vorgenannten Schriften und in der US 2006/0161019 werden solche (insbesondere ringförmigen) Katalysatorformkörper insbesondere für eine heterogen katalysierte partielle Vorgängergasphasenoxidation von Propylen zu Acrolein bzw. Acrolein und Acrylsäure sowie von iso-Buten zu Methacrolein bzw. Methacrolein und Methacrylsäure empfohlen. Die EP-A 970942 empfiehlt solche Katalysatorformkörper vor allem für eine heterogen katalysierte Ammoxidation von Propylen zu Acrylnitril bzw. von iso-Buten zu Methacrylnitril als Vorgängergasphasenpartialoxidation.

Eine für eine der vorgenannten Vorgängergasphasenpartialoxidationen besonders relevante Ringgeometrie im Fall von Multielementoxid (I)-Vollkatalysatorformkörpern ist z. B. die Geometrie A (Außendurchmesser) x I (Innendurchmesser) x B (Länge, Höhe) = 5 mm x 2 mm x 3 mm.

Andere für Vorgängergasphasenpartialoxidationen günstige Multimetalloxid (I)-Vollkatalysator-Ringgeometrien A x I x B sind die Geometrien 5 mm x 2 mm x 2 mm, oder 5 mm x 3 mm x 3 mm, oder 5,5 mm x 3,5 mm x 3 mm, oder 6 mm x 4 mm x 3 mm, oder 6,5 mm x 4,5 mm x 3 mm, oder 7 mm x 5 mm x 3 mm, oder 7 mm x 3 mm x 7 mm, oder 7 mm x 4 mm x 7 mm.

Betreffend die Aktivmassen der Stöchiometrie der allgemeinen Formel I betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient f vorteilhaft 0,5 oder 1 bis 10.

Besonders bevorzugt liegen die vorgenannten stöchiometrischen Koeffizienten gleichzeitig in den vorgenannten Vorzugsbereichen.

Ferner ist X¹ vorzugsweise Kobalt, X² ist vorzugsweise K, Cs und/oder Sr, besonders bevorzugt K, X³ ist bevorzugt Wolfram, Zink und/oder Phosphor und X⁴ ist bevorzugt Si. Besonders bevorzugt weisen die Variablen X¹ bis X⁴ gleichzeitig die vorgenannten Bedeutungen auf.

Alles in dieser Schrift Gesagte ist zudem insbesondere dann zutreffend, wenn das Vorgängerkatalysatorfestbett wenigsten Katalysatorformkörper umfasst, deren Aktivmasse ein Multielementoxid der allgemeinen Formel II,

Mo₁₂PₐV_{b}X_{c}¹X_{d}²Xₑ³Sb_{f}Re_{g}SₕOₙ (II),

mit
- X¹ =: Kalium, Rubidium und/oder Cäsium,
- X² =: Kupfer und/oder Silber,
- X³ =: Cer, Bor, Zirkonium, Mangan, Niob und/oder Wismut,
- a =: 0,5 bis 3
- b =: 0,01 bis 3,
- c =: 0 bis oder 0,2 bis 3,
- d =: 0 bis oder 0,01 bis 2,
- e =: 0 bis 2,
- f =: 0 bis oder 0,01 bis 2,
- g =: 0 bis 1,
- h =: 0 bis oder 0,001 bis 0,5, und
- n =: eine Zahl, die durch die Wertigkeit und Häufigkeit der von Sauerstoff verschie denen Elemente in II bestimmt wird,
ist.

Alles in dieser Schrift Gesagte ist insbesondere dann zutreffend, wenn das Vorgängerkatalysatorfestbett zu wenigstens 25 %, oder zu wenigstens 50 %, oder zu wenigstens 75 % seines Gewichtes oder nur aus Katalysatorformkörpern besteht, deren Aktivmasse ein Multielementoxid II ist. Katalysatorformkörper mit einem Multielementoxid II als Aktivmasse eignen sich insbesondere für eine heterogen katalysierte partielle Vorgängergasphasenoxidation von organischen C₄- Verbindungen (z. B. von n-Butan zu Maleinsäureanhydrid) und ganz besonders für eine solche von Methacrolein zu Methacrylsäure. Dabei kann z. B. wie in der EP-A 467144 sowie wie in der DE-A 102007005606 beschrieben verfahren werden (sowie wie in dem in diesen beiden Schriften zitierten Stand der Technik).

Die Herstellung von Multielementoxid-II-Katalysatorformkörpern findet sich ebenfalls in den beiden vorgenannten Schriften. Besonders bevorzugt sind für eine heterogen katalysierte partielle Vorgängergasphasenoxidation von Methacrolein zu Methacrylsäure ringförmige Vollkatalysatorformkörper mit A x I x B = 7 mm x 3 mm x 6,9 mm (oder x 7,0 mm).

Alles in dieser Schrift Gesagte ist ferner insbesondere dann zutreffend, wenn das Vorgängerkatalysatorfestbett wenigstens Katalysatorformkörper umfasst, deren Aktivmasse ein Multielementoxid der allgemeinen Formel III,

V₁P_{b}Mo_{c}X_{d}¹Xₑ²Oₙ (III),

mit
- X¹ =: wenigstens ein Element aus der Gruppe bestehend aus Fe, Bi, Co, Ni, Si, Zn, Hf, Zr, Ti, Cr, Mn, Cu, B, Sn und Nb,
- X² =: K, Na, Rb, Cs und/oder Tl,
- b =: 0,9 bis 1,5,
- c =: > 0 bis 0,2, vorzugsweise 0,0001 bis 0,1,
- d =: 0 bis 0,1,
- e =: 0 bis 0,1, und
- n =: eine Zahl, die durch die Wertigkeit und Häufigkeit der von Sauerstoff verschiedenen Elemente in III bestimmt wird,
ist.

Alles in dieser Schrift Gesagte ist insbesondere dann zutreffend, wenn das Vorgängerkatalysatorfestbett zu wenigstens 25 %, oder zu wenigstens 50 %, oder zu wenigstens 75 % seines Gewichtes, oder nur aus Katalysatorformkörpern besteht, dessen Aktivmasse ein Multielementoxid III ist.

Katalysatorformkörper mit einem Multielementoxid III als Aktivmasse eignen sich insbesondere für die heterogen katalysierte partielle Gasphasenoxidation von Kohlenwasserstoffen mit mindestens vier Kohlenstoffatomen (insbesondere n-Butan, n-Butene und/oder Benzol) zu Maleinsäureanhydrid als Vorgängergasphasenpartialoxidation.

Mit Vorteil handelt es sich dabei um ringförmige Vollkatalysatoren. Günstige Ringgeometrien sind dabei z. B. A x I x B = 6,6 mm x 3,7 mm x 4,2 mm oder 5 mm x 2,5 mm x 3,2 mm. Im übrigen sind die in den Schriften US-A 5011945, WO1997/012674, WO 03/078310, WO 01/68245, DE-A 102005035978 sowie DE-A 102007005606 empfohlenen Partialoxidationsverfahrensbedingungen anwendbar. Vorgenannte Schriften beschreiben auch die Herstellung von Multielementoxid (III)-Katalysatorformkörpern. Weiterhin ist alles in dieser Schrift Gesagte insbesondere dann zutreffend, wenn das Vorgängerkatalysatorbett wenigstens einen Katalysatorformkörper umfasst, dessen Aktivmasse ein Multielementoxid der allgemeinen Formel IV,

Mo₁₂VₐX_{b}¹X_{c}²X_{d}³Xₑ⁴X_{f}⁵X_{g}⁶Oₙ (IV),

mit
- X¹ =: W, Nb, Ta, Cr und/oder Ce,
- X² =: Cu, Ni, Co, Fe, Mn und/oder Zn,
- X³ =: Sb und/oder Bi,
- X⁴ =: eines oder mehrere Alkalimetalle (Li, Na, K, Rb, Cs) und/oder H,
- X⁵ =: eines oder mehrere Erdalkalimetalle (Mg, Ca, Sr, Ba),
- X⁶ =: Si, Al, Ti und/oder Zr,
- a =: 1 bis 6,
- b =: 0,2 bis 4,
- c =: 0 bis 18, vorzugsweise 0,5 bis 18,
- d =: 0 bis 40,
- e =: 0 bis 2,
- f =: 0 bis 4,
- g =: 0 bis 40 und
- n =: eine Zahl, die durch die Wertigkeit und Häufigkeit der von Sauerstoff verschie denen Elemente in IV bestimmt wird,
ist.

Alles in dieser Schrift Gesagte ist insbesondere dann zutreffend, wenn das Vorgängerkatalysatorfestbett zu wenigstens 25 %, oder zu wenigstens 50 %, oder zu wenigstens 75 % seines Gewichtes, oder nur aus Katalysatorformkörpern besteht, deren Aktivmasse ein Multielementoxid IV ist.

Katalysatorformkörper mit einem Multielementoxid IV als Aktivmasse eignen sich insbesondere für die heterogen katalysierte partielle Gasphasenoxidation von Acrolein zu Acrylsäure als Vorgängergasphasenpartialoxidation. Mit Vorteil sind hier die Katalysatorformkörper Schalenkatalysatoren (insbesondere ringförmige), wie sie z. B. gemäß der DE-A 102004025445, der DE-A 10350822, der DE-A 102007010422, der US 2006/0205978, der EP-A 714700 und dem Deutschen Aktenzeichen 102007010422.9 und dem in diesen Schriften zitierten Stand der Technik erhältlich sind. Die Aktivmassenschalendicke kann 10 bis 1000 µm, bevorzugt 50 bis 500 µm und besonders bevorzugt 150 bis 250 µm betragen. Günstig sind hier vor allem die Schalendicken der beispielhaften Ausführungsformen der EP-A 714700. Bevorzugte Ringgeometrie ist diejenige mit A x I x B = 7 mm x 4 mm x 3 mm. Vorgenannte Schriften und der in diesen Schriften zitierte Stand der Technik beschreiben auch die Partialoxidationsverfahrensbedingungen.

Außerdem eignet sich das in dieser Schrift Gesagte insbesondere dann, wenn das Vorgängerkatalysatorfestbett wenigstens Katalysatorformkörper umfasst, deren Multielementoxid ein Multielementoxid ist, das als von Sauerstoff verschiedene Elemente neben den Elementen Mo und V wenigstens eines der beiden Elemente Te und Sb, und wenigstens eines der Elemente aus der Gruppe umfassend Nb, Pb, Ta, W, Ti, Al, Zr, Cr, Mn, Ga, Fe, Ru, Co, Rh, Ni, Pd, Pt, La, Bi, B, Ce, Sn, Zn, Si, Na, Li, K, Mg, Ag, Au und In in Kombination enthalten (dabei kann der Gewichtsanteil solcher Katalysatorformkörper am Katalysatorfestbett wenigstens 25 %, oder wenigstens 50 %, oder wenigstens 75 %, oder auch 100 % des Gewichtes des Katalysatorfestbetts betragen).

Ihre Herstellung kann z. B. wie auf Seiten 25, 26 der WO 2004/108267 beschrieben erfolgen.
Bevorzugt enthält die Kombination dabei aus der letzten Elementgruppe die Elemente Nb, Ta, W und/oder Ti und besonders bevorzugt das Element Nb.

Bevorzugt enthalten die vorgenannten Multielementoxidmassen die vorgenannte Elementkombination in der Stöchiometrie V,

Mo₁V_{b}M_{c}¹M_{d}² (V),

mit
- M¹ =: Te und/oder Sb,
- M² =: wenigstens eines der Elemente aus der Gruppe umfassend Nb, Ta, W, Ti, Al, Zr,Cr, Mn, Ga, Fe, Ru, Co, Rh, Ni, Pd, Pt, La,Bi, Ce, Sn, Zn, Si, Na, Li, K, Mg, Ag, Au und In,
- b =: 0,01 bis 1,
- c =: > 0 bis 1, und
- d =: > 0 bis 1.

Bevorzugt ist M¹ = Te und M² = Nb, Ta, W und/oder Ti. Vorzugsweise ist M² = Nb. Der stöchiometrische Koeffizient b beträgt mit Vorteil 0,1 bis 0,6. In entsprechender Weise beläuft sich der Vorzugsbereich für den stöchiometischen Koeffizienten c auf 0,01 bis 1 bzw. auf 0,05 bis 0,4 und günstige Werte für d betragen 0,01 bis 1 bzw. 0,1 bis 0,6. Besonders günstig ist es, wenn die stöchiometrischen Koeffizienten b, c und d simultan in den vorgenannten Vorzugsbereichen liegen. Das Vorgenannte gilt insbesondere dann, wenn die Aktivmasse hinsichtlich ihrer von Sauerstoff verschiedenen Elemente aus einer vorgenannten Elementkombination besteht.

Dies sind dann insbesondere die Multielementoxidaktivmassen der allgemeinen Stöchiometrie VI

Mo₁V_{b}M_{c}¹M_{d}²Oₙ (VI),

wobei die Variablen die bezüglich der Stöchiometrie V angeführte Bedeutung aufweisen und n = eine Zahl ist, die durch die Wertigkeit und Häufigkeit der von Sauerstoff verschiedenen Elemente in (VI) bestimmt wird.

Die Herstellung derartiger Multielementoxidaktivmassen und der aus ihnen gefertigten Katalysatorformkörper findet sich z. B. in den Schriften DE-A 19835247, DE-A 10122027, DE-A 10051419, DE-A 10046672, DE-A 10122027 und DE-A 10119933.

Sie eignen sich u. a. als Katalysatoren für die heterogen katalysierte partielle Gasphasenoxidation von Acrolein zu Acrylsäure, von Propan zu Acrolein und/oder Acrylsäure, von iso-Butan zu Methacrolein und/oder Methacrylsäure sowie für die Ammoxidation von Propan zu Acrylnitril sowie von iso-Butan zu Methacrylnitril als Vorgängergasphasenpartialoxidation (vgl. z. B. Deutsches Aktenzeichen 102007010422.9, EP-A 1734030, EP-A 93846, EP-A 608838, EP-A 895809, DE-A 10122027, DE-A 19835247, EP-A 1254709, EP-A 1192987, EP-A 962253, EP-A 1262235, DE-A 10119933, DE-A 10051419, DE-A 10034825, DE-A 10046672, DE-A 10254278, DE-A 10033121, DE-A 10029338, DE-A 10248584).

Im besonderen erweist sich das erfindungsgemäße Verfahren dann als geeignet, wenn das Vorgängerkatalysatorfestbett wenigstens Katalysatorformkörper umfasst, deren Aktivmasse ein Gemisch aus einem das Element Mo in oxidiertem Zustand enthaltenden Multielementoxid (z. B. einem solchen der allgemeinen Formeln (I) bis (VI)) und wenigstens einer feinteiligen Substanz S ausgewählt aus der Gruppe bestehend aus Oxiden des Molybdäns und aus Verbindungen des Molybdäns, aus denen sich unter der Einwirkung von erhöhter Temperatur und molekularem Sauerstoff ein Oxid des Molybdäns bildet, ist. Derartige Katalysatorformkörper zeichnen sich bei Vorgängergasphasenpartialoxidationen in der Regel durch eine verbesserte Standzeit aus (vgl. z. B. Deutsches Aktenzeichen 10 2007 010 422.9).

Dabei kann das Vorgängerkatalysatorfestbett zu wenigstens 25 %, oder zu wenigstens 50 %, oder zu wenigstens 75 %, oder zu 100 % seines Gewichts aus solchen Katalysatorformkörpern bestehen.

Grundsätzlich kann beim erfindungsgemäßen Verfahren die neue heterogen katalysierte partielle Gasphasenoxidation von der Vorgängergasphasenpartialoxidation verschieden sein.

Das gleiche gilt für das Vorgängerkatalysatorfestbett und das neue Katalysatorfestbett. Ebenso muss die Aktivmasse der Katalysatorformkörper des neuen Katalysatorfestbetts nicht in notwendiger Weise Mo enthalten.

Im Regelfall wird die neue heterogen katalysierte partielle Gasphasenoxidation beim erfindungsgemäßen Verfahren jedoch die gleiche Gasphasenpartialoxidation (oder eine ähnliche) wie die Vorgängergasphasenpartialoxidation sein. In diesem Fall gilt alles in dieser Schrift bezüglich der Vorgängergasphasenpartialoxidation Gesagte in entsprechender Weise auch für die neue heterogen katalysierte partielle Gasphasenoxidation und das neue Katalysatorfestbett kann in diesem Fall von der gleichen Art wie das Vorgängerkatalysatorfestbett sein. Dieser Sachverhalt ist üblicherweise dadurch bedingt, dass ein Rohrbündelreaktor in vielen Fällen in seiner Detailausgestaltung auf eine bestimmte heterogen katalysierte Vorgängergasphasenpartialoxidation abgestimmt ausgelegt wird.

Grundsätzlich kommt beim erfindungsgemäßen Verfahren als neue heterogen katalysierte partielle Gasphasenoxidation einer organischen Ausgangsverbindung aber jedwede heterogen katalysierte partielle Gasphasenoxidation einer organischen Ausgangsverbindung (unabhängig von der Vorgängergasphasenpartialoxidation) in Betracht.

Als solche heterogen katalysierte partielle Oxidationen einer organischen Ausgangsverbindung seien beispielhaft genannt die Umsetzung von Methanol zu Formaldehyd (vgl. z. B. CH-A 449600, CH-A 38828), die Umsetzung von Propen zu Acrolein und/oder Acrylsäure (vgl. z. B. die DE-A 23 51 151), die Umsetzung von tert.-Butanol, iso-Buten, iso-Butan, iso-Butyraldehyd oder dem Methylether des tert.-Butanols zu Methacrolein und/oder Methacrylsäure (vgl. z. B. DE-A 25 26 238, EP-A 092 097, EP-A 058 927, DE-A 41 32 263, DE-A 41 32 684 und DE-A 40 22 212), die Umsetzung von Acrolein zu Acrylsäure, die Umsetzung von Methacrolein zu Methacrylsäure (vgl. z. B. DE-A 25 26 238), die Umsetzung von o-Xylol oder Naphthalin zu Phthalsäureanhydrid (vgl. z. B. EP-A 522 871 sowie die Umsetzung von Butadien zu Maleinsäureanhydrid (vgl. z. B. DE-A 21 06 796 und DE-A 16 24 921), die Umsetzung von C₄-Kohlenwasserstoffen (insbesondere 1-Buten, 2-Buten, Butadien und/oder n-Butan) zu Maleinsäureanhydrid (vgl. z. B. GB-A 1 464 198 und GB-A 1 291 354), die Umsetzung von Indanen zu Anthrachionen (vgl. z. B. DE-A 20 25 430), die Umsetzung von Ethylen zu Ethylenoxid (vgl. z. B. EP-A 352 849, EP-A 352 850, EP-A 532 325, US-A 5,155,242 und US-A 5,262,551) oder von Propylen zu Propylenoxid (vgl. z. B. DE-AS 12 54 137, DE-A 21 59 346, EP-A 372 972, WO 89/07101, DE-A 43 11 608), die Umsetzung von Propylen und/oder Acrolein zu Acrylnitril (vgl. z. B. DE-A 23 51 151), die Umsetzung von iso-Buten und/oder Methacrolein zu Methacrylnitril (d. h., der Begriff der partiellen Oxidation soll in dieser Schrift auch die partielle Ammoxidation, d. h. eine partielle Oxidation im Beisein von Ammoniak, umfassen), die oxidative Dehydrierung von Kohlenwasserstoffen (vgl. z. B. DE-A 23 51 151), die Umsetzung von Propan zu Acrylnitril oder zu Acrolein und/oder Acrylsäure (vgl. z. B. DE-A 101 31 297, EP-A 1 090 684, EP-A 608 838, DE-A 100 46 672, EP-A 529 853, WO 01/96270 und DE-A 100 28 582) etc..

Die Anmeldung umfasst daher auch die Durchführung von heterogen katalysierten partiellen Gasphasenoxidationen von organischen Ausgangsverbindungen in nach einem erfindungsgemäßen Verfahren wieder beschickten Rohrbündelreaktoren Dies gilt insbesondere dann, wenn die Gasphasenpartialoxidation eine der Vorgenannten ist.

Abschließend sei nochmals festgehalten, dass sich das erfindungsgemäße Verfahren vor allem dann eignet, wenn die heterogen katalysierte partielle Vorgängergasphasenoxidation eine solche von Propylen zu Acrolein und/oder Acrylsäure, oder von Acrolein zu Acrylsäure, oder von iso-Buten zu Methacrolein und/oder Methacrylsäure, oder von Methacrolein zu Methacrylsäure, oder von Propan zu Acrolein und/oder Acrylsäure, oder von iso-Butan zu Methacrolein und/oder Methacrylsäure, oder von Propylen zu Acrylnitril, oder von Propan zu Acrylnitril, oder von iso-Buten zu Methacryllnitril, oder von iso-Butan zu Methacrylnitril, oder von einem oder mehreren C₄-Kohlenwasserstoffen zu Maleinsäureanhydrid, oder von Methanol zu Formaldehyd ist. Dies inbesondere dann, wenn das Katalysatorfestbett eine der Multielementoxidaktivmassen der allgemeinen Formeln I bis VI umfasst.

Das erfindungsgemäße Verfahren ermöglicht über vieljährige Betriebsdauern von Rohrbündelreaktoren die reproduzierbare Wiederbeschickung ihrer Reaktionsrohre (mit größtmöglicher Einheitlichkeit der Beschickung über alle Reaktionsrohre) und auf dieser Grundlage die Zielproduktherstellung mit maximaler Selektivität der Zielproduktbildung.

Das erfindungsgemäße Verfahren ist damit nicht zuletzt auf ein Verfahren zur Nachsorge einer heterogen katalysierten partiellen Gasphasenoxidation einer organischen Verbindung, die im in den Reaktionsrohren eines Rohrbündelreaktors befindlichen Katalysatorfestbett durchgeführt worden ist, wobei das Katalysatorfestbett wenigstens Katalysatorformkörper umfasst, deren Aktivmasse ein das Element Mo in oxidiertem Zustand enthaltendes Multielementoxid ist und ein Wasserdampf enthaltendes Produktgasgemisch erzeugt wurde, gerichtet, das dadurch gekennzeichnet ist, dass man das Katalysatorfestbett aus den Reaktionsrohren des Rohrbündelreaktors entnimmt und wenigstens bei einem Teil der Reaktionsrohre den auf deren Innenwand abgeschiedenen, Molybdänoxid und/oder Molybdänoxidhydrat enthaltenden, Feststoffbelag mit Hilfe einer Bürste wenigstens teilweise wegbürstet. Im übrigen kann sowohl die Beschickung der Reaktionsrohre mit Vorgängerkatalysatorfestbett als auch mit neuem Katalysatorfestbett wie in dem Deutschen Aktenzeichen 102007017080.9 beschrieben durchgeführt werden.

Grundsätzlich ist das erfindungsmäßige Verfahren auch dann anwendbar, wenn zwar das Vorgängerkatalysatorfestbett keine Katalysatorformkörper umfasst, deren Aktivmasse ein das Element Mo in oxidiertem Zustand enthaltendes Multielementoxid ist, das Reaktionsgaseingangsgemisch der partiellen Vorgängergasphasenoxidation aber das Wasserdampf enthaltende Produktgasgemisch einer vorgeschalteten heterogen katalysierten partiellen Gasphasenoxidation einer organischen Verbindung umfasst, deren Katalysatorfestbett Katalysatorformkörper umfasst, deren Aktivmasse ein das Element Mo in oxidiertem Zustand enthaltendes Multielementoxid ist.

### Beispiel 1

Ausführungsbeispiel an Hand einer zweistufigen heterogen katalysierten partiellen Gasphasenoxidation von Propylen zu Acrylsäure in zwei hintereinander angeordneten Einzonenrohrbündelreaktoren.

Beide Rohrbündelreaktoren waren mit frischem Katalysatorfestbett beschickt. Nach beendeter Formierung der Katalysatorfestbetten waren die stationären Betriebsbedingungen wie folgt:
A) Beschreibung der allgemeinen Verfahrensbedingungen

### I. Die erste Reaktionsstufe

| | |
|---|---|
| Verwendetes Wärmeaustauschmittel: | Salzschmelze, bestehend aus 60 Gew.-% Kaliumnitrat und 40 Gew.-% Natriumnitrit. |
| Material der Reaktionsrohre: | ferritischer Stahl der DIN Werkstoffnummer 1.0481. |
| Abmessungen der Reaktionsrohre: | 3200 mm Länge; |
| | 25 mm Innendurchmesser; |
| | 30 mm Außendurchmesser (Wandstärke: 2,5 mm). |
| Anzahl der Reaktionsrohre im Rohrbündel: | 25500. |
| Reaktor: | Zylinderförmiger Behälter (ferritischer Stahl der DIN Werkstoffnummer 1.0345) eines Außendurchmessers von 6800 mm; Mantelwanddicke = 1,8 cm im Mittelteil, oben und unten auf 2,5 cm verstärkt; ringförmig vertikal angeordnetes Rohrbündel mit einem freien zentralen Raum. |
| | Durchmesser des zentralen freien Raumes: 1000 mm. Abstand der am weitesten außen liegenden Reaktionsrohre zur Behälterwand: 150 mm. Homogene Reaktionsrohrverteilung im Rohrbündel (6 äquidistante Nachbarrohre pro Reaktionsrohr). |
| | Reaktionsrohrteilung: 38 mm. |
| | Die Reaktionsrohre waren mit ihren Enden in Öffnungen von Rohrböden der Bodendicke 125 mm eingedichtet befestigt und mündeten mit ihren Öffnungen in eine am oberen Ende mit dem Behälter verbundene und den oberen Reaktorboden überspannende Reaktorhaube und am unteren Ende in den zylindrischen Übergang zum Nachkühler. |
| | Die den oberen Reaktorboden (den Reaktorboden E*) überspannende Reaktorhaube wies eine Öffnung E* (in Form eines Gaseintrittsstutzens) mit einem Durchmesser von 1020 mm auf. |
| | Die Rohrböden und die anderen Elemente des Rohrbündelreaktors waren aus ferritischem Stahl der DIN Werkstoffnummer 1.0481 gefertigt. In die Reaktorbodenoberfläche E* (am äußersten Reaktionsrohrkreis) und in die obere Reaktorhaube (die Reaktorhaube E*) war jeweils ein Thermoelement eingelassen bzw. eingeführt. Die obere Reaktorhaube (Gesamtwandstärke = 20 mm) war innen mit Edelstahl vom Typ 1.4571 (nach DIN EN 10020) plattiert (Plattierungsstärke: 3 mm). |
| | Zuführung des Wärmeaustauschmittels zum Rohrbündel: Das Rohrbündel war durch drei zwischen den Rohrböden längs derselben aufeinanderfolgend angebrachte Umlenkscheiben (Dicke jeweils 10 mm) in 4 äquidistante (jeweils 730 mm) Längsabschnitte (Zonen) geteilt. |

Die unterste und die oberste Umlenkscheibe wies Ringgeometrie auf, wobei der Ringinnendurchmesser 1000 mm betrug und der Ringaußendurchmesser sich bis zur Behälterwand eingedichtet (abdichtend) erstreckte. Die Reaktionsrohre waren an den Umlenkscheiben nicht abdichtend (nicht eingedichtet) befestigt. Vielmehr waren eine Spaltbreite < 0,5 mm aufweisende Spalte so belassen, dass die Querströmungsgeschwindigkeit der Salzschmelze innerhalb einer Zone möglichst konstant war.

Die mittlere Umlenkscheibe war kreisförmig und erstreckte sich bis zu den am weitesten außen liegenden Reaktionsrohren des Rohrbündels.

Die Kreisführung der Salzschmelze wurde durch zwei Salzpumpen bewerkstelligt, von denen jede eine Rohrbündellängshälfte versorgte.

Die Pumpen drückten die Salzschmelze in einen um den Reaktormantel unten angebrachten Ringkanal, der die Salzschmelze über den Behälterumfang verteilte. Durch im Reaktormantel befindliche Fenster gelangte die Salzschmelze im untersten Längsabschnitt zum Rohrbündel. Die Salzschmelze floss dann der Vorgabe der Umlenkbleche folgend in der Abfolge
- von außen nach innen,
- von innen nach außen,
- von außen nach innen,
- von innen nach außen,
im wesentlichen mäanderförmig, über den Behälter betrachtet, von unten nach oben. Durch im obersten Längsabschnitt um den Behälterumfang angebrachte Fenster sammelte sich die Salzschmelze (verließ die Salzschmelze mit der Temperatur T_{w}^{1,aus} den Reaktionsrohrumgebungsraum) in einem oberen um den Reaktormantel angebrachten Ringkanal und wurde nach Abkühlung auf die ursprüngliche Eintrittstemperatur T_{w}^{1,ein} durch die Pumpen wieder in den unteren Ringkanal gedrückt.

Das Reaktionsgaseintrittsgemisch 1 war ein Gemisch aus Luft, chemical grade Propylen und Kreisgas (Kreisgas ist das Gas, das nach der Zielproduktabtrennung aus dem Produktgasgemisch der zweistufigen heterogen katalysierten Partialoxidation verbleibt).

| | |
|---|---|
| Reaktorbeschickung: | Salzschmelze und Reaktionsgasgemisch wurden über den Reaktor betrachtet im Gegenstrom geführt. Die Salzschmelze trat unten ein, das Reaktionsgasgemisch über die Öffnung E* oben. Die Eintrittstemperatur der Salzschmelze betrug T_{w}^{1,ein} |
| | Die Austrittstemperatur der Salzschmelze lag bei T_{w}^{1,aus}. |
| | T_{w}^{1,aus} - T_{w}^{1,ein} betrug > 0 und ≤ 2 °C. |
| | Die Pumpleistung betrug 6200 m³ Salzschmelze/h. Das Reaktionsgaseintrittsgemisch 1 wurde dem Reaktor mit einer Temperatur von T_{G}^{E*,1} bei Durchtritt durch die Öffnung E* zugeführt. |
| Propylenbelastung des Katalysatorfestbetts 1: | Sie betrug L¹ NI/(l•h). |
| | |
| Reaktionsrohrbeschickung mit Katalysatorfestbett 1 (von oben nach unten): | Zone A: 50 cm |
| | Vorschüttung aus Steatit-Ringen der Geometrie 7 mm x 3 mm x 4 mm (Außendurchmesser x Länge x Innendurchmesser) |
| | |
| | Zone B: 100 cm |
| | Katalysatorbeschickung mit einem homogenen Gemisch aus 30 Gew.-% an Steatit-Ringen der Geometrie 5 mm x 3 mm x 2 mm (Außendurchmesser x Länge x Innendurchmesser) und 70 Gew.-% eines ringförmigen Vollkatalysators, der wie der Vollkatalysator BVK 3 aus der WO 2005/030393 unter Verwendung von TIMREX T 44 der Fa. Timcal AG (CH-Bodio) als Hilfsgraphit hergestellt wurde und ohne Berücksichtigung von noch enthaltenem Graphit die Stöchiometrie Mo₁₂Bi₁W₂Co_{5,5}Fe_{2,94}Si_{1,59}K_{0,08}Oₓ aufwies, mit der Ringgeometrie A x L x I = 5 mm x 3 mm x 2 mm. |
| | |
| | Zone C: 170 cm |
| | Katalysatorbeschickung nur mit dem für die Zone B verwendeten ringförmigen (5 mm x 3 mm x 2 mm = Außendurchmesser x Länge x Innendurchmesser) Vollkatalysator. |
| | |
| Thermorohre (ihre Anzahl belief sich auf 10, die im zentralen Bereich des Rohrbündels gleichmäßig verteilt waren) waren wie folgt gestaltet und beschickt, um die Temperatur in den Reaktionsrohren repräsentativ zu überwachen. | |
| | |
| | Jedes der 10 Thermorohre wies eine zentrale Thermohülle mit 40 Temperaturmessstellen auf (d.h., jedes Thermorohr enthielt 40 Thermoelemente, die mit unterschiedlicher Länge in eine Thermohülse integriert waren und so ein Multithermoelement bildeten, mit dem innerhalb des Thermorohres auf unterschiedlichen Höhen simultan die Temperatur ermittelt werden konnte). |
| | 20 der jeweils 40 Temperaturmessstellen befanden sich im Bereich des ersten Meters des aktiven Abschnitts des Katalysatorfestbetts (in Strömungsrichtung des Reaktionsgasgemischs). |
| | |
| | Der Innendurchmesser eines Thermorohres betrug 29 mm. Die Wanddicke und das Rohrmaterial war wie bei den Arbeitsrohren beschaffen. |
| | |
| | Der Außendurchmesser der Thermohülse betrug 10 mm. |
| | |
| | Die Befüllung der Thermorohre erfolgte wie folgt: |
| | |
| | Ein Thermorohr wurde mit dem ringförmigen Vollkatalysator aus Zone B befüllt. Zusätzlich wurde in das Thermorohr aus dem ringförmigen Vollkatalysator erzeugter Katalysatorsplit der Längstausdehnung 0,5 bis 5 mm beigefüllt. |
| | |
| | Die Beifüllung des Katalysatorsplits erfolgte über den gesamten aktiven Abschnitt des Katalysatorfestbetts des jeweiligen Thermorohrs homogen verteilt so, dass der Druckverlust des Reaktionsgasgemischs beim Durchgang durch das Thermorohr demjenigen beim Durchgang des Reaktionsgasgemischs durch ein Arbeitsrohr entsprach (bezogen auf den aktiven Abschnitt des Katalysatorfestbetts (d.h., die Inertabschnitte ausgenommen) im Thermorohr waren dazu 5 bis 30 Gew.-% an Katalysatorsplit erforderlich). Gleichzeitig war die jeweilige Gesamtfüllhöhe von Aktiv- und Inertabschnitten in den Arbeits- und Thermorohren gleich bemessen und das Verhältnis von im Rohr enthaltener Gesamtmenge an Aktivmasse zu Wärmeübergangsfläche des Rohres bei Arbeits- und Thermorohren auf den im wesentlichen selben Wert eingestellt. |

### II. Die Zwischenkühlung

Das die erste Reaktionsstufe mit einer der Salzschmelzeeintrittstemperatur T_{w}^{1,ein} entsprechenden Temperatur verlassende Acrolein enthaltende Produktgasgemisch 1 wurde zum Zweck der Zwischenkühlung durch einen mit einer Salzschmelze aus 60 Gew.-% Kaliumnitrat und 40 Gew.-% Natriumnitrit gekühlten Einzonen-Rohrbündelwärmeaustauscher aus ferritischem Stahl geführt, der unmittelbar an den unteren Rohrboden des Rohrbündelreaktors der ersten Reaktionsstufe angeflanscht war. Der Abstand des unteren Rohrbodens des Rohrbündelreaktors zum oberen Rohrboden des Kühlers betrug dabei 10 cm. Die Salzschmelze und das Produktgasgemisch wurden dabei über den Wärmeaustauscher betrachtet im Gegenstrom geführt. Das Salzbad selbst floss in gleicher Weise wie im Erststufen-Einzonen-Rohrbündel-Festbettreaktor mäanderförmig um die Kühlrohre, durch die das Produktgasgemisch 1 geleitet wurde. Die Länge der Kühlrohre betrug 1,65 m, ihr Innendurchmesser lag bei 2,6 cm und ihre Wanddicke war 2,5 mm. Die Anzahl der Kühlrohre belief sich auf 8000. Der Außendurchmesser des Wärmetauschers betrug 6,8 m, die Wanddicke entsprach dem des Reaktors.

Sie waren mit einheitlicher Rohrteilung gleichmäßig über den Querschnitt verteilt.

In den Eingang der Kühlrohre (in Strömungsrichtung) waren Spiralen aus Edelstahl eingeführt, deren Querschnitt nahezu dem der Kühlrohre entsprach. Ihre Länge betrug 700 mm bis 1000 mm (alternativ können die Kühlrohre mit großen Inertmaterialringen befüllt werden). Sie dienten der Verbesserung des Wärmeübergangs.

Das Acrolein enthaltende Produktgasgemisch 1 verließ den Zwischenkühler mit einer Temperatur T_{G}^{Z,aus}. Anschließend wurde ihm komprimierte Luft (Sekundärluft), die eine Temperatur von 140 °C aufwies, in einer solchen Menge zugemischt, dass der Sauerstoffgehalt im Produktgasgemisch 2 3,0 Vol.-% betrug, woraus die Zusammensetzung des Reaktionsgaseintrittsgemischs 2 für die zweite Reaktionsstufe resultierte.

Dieses wurde mit seiner Temperatur T_{G}^{E,2} in die Öffnung E der oberen Reaktorhaube des Einzonen-Rohrbündelrohr-Festbettreaktors der zweiten Reaktionsstufe zugeführt.

### III. Die zweite Reaktionsstufe

Es wurde ein Einzonen-Rohrbündel-Festbettreaktor verwendet, der mit jenem der ersten Stufe baugleich war, jedoch eine obere und eine untere Reaktorhaube aufwies. Sein oberer Reaktorboden ist der Reaktorboden E.
Die Zusammensetzung des Reaktionsgaseintrittsgemischs 2 bestand aus dem Produktgasgemisch der ersten Reaktionsstufe und der Sekundärluft.

| | |
|---|---|
| Reaktorbeschickung: | Salzschmelze und Reaktionsgasgemisch wurden über den Reaktor betrachtet im Gegenstrom geführt. Die Salzschmelze trat unten ein, das Reaktionsgasgemisch oben. |
| | |
| | Die Eintrittstemperatur der Salzschmelze betrug T_{w}^{2,ein}. Ihre Austrittstemperatur lag bei T_{w}^{2,aus}. |
| | T_{w}^{2,aus} - T_{w}^{2,ein} betrug > 0 und ≤ 2 °C. |
| | |
| | Die Pumpleistung betrug 6200 m³ Salzschmelze/h. |
| | |
| | Das Reaktionsgaseintrittsgemisch 2 wurde dem Reaktor mit einer Temperatur von T_{G}^{E,2} bei Durchtritt durch die Öffnung E zugeführt. |
| | |
| Die Acroleinbelastung des Katalysatorfestbetts 2: | Sie betrug L² NI/(l•h). |
| Die Reaktionsrohrbeschickung mit Katalysatorfestbett 2 (von oben nach unten) war: Zone A: | |
| | 20 cm Vorschüttung aus Steatit-Ringen der Geometrie 7 mm x 3 mm x 4 mm (Außendurchmesser x Länge x Innendurchmesser). |
| | |
| | Zone B: |
| | 100 cm Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% des ringförmigen (ca. 7 mm x 3 mm x 4 mm) Schalenkatalysators K_{A} aus der DE 10 2004 025 445 mit der Aktivmasse Mo₁₂V₃W_{1,2}Cu_{2,4}Oₓ und einem Aktivmassenanteil von 20 Gew.-%. |
| | |
| | Zone C: |
| | 200 cm Katalysatorbeschickung mit dem ringförmigen (ca. 7 mm x 3 mm x 4 mm) Schalenkatalysator aus der Zone B. |
| Thermorohre (ihre Anzahl belief sich auf 10, die im zentralen Bereich des Rohrbündels gleichmäßig verteilt waren) waren wie folgt gestaltet und beschickt, um die Temperatur in den Reaktionsrohren repräsentativ zu überwachen. | |
| | Jedes der 10 Thermorohre wies eine zentrale Thermohülse mit 40 Temperaturmessstellen auf (d.h., jedes Thermorohr enthielt 40 Thermoelemente, die mit unterschiedlicher Länge in eine Thermohülse integriert waren und so ein Multithermoelement bildeten, mit dem innerhalb des Thermorohres auf unterschiedlichen Höhen simultan die Temperatur ermittelt werden konnte). |
| | |
| | 20 der jeweils 40 Temperaturmessstellen befanden sich im Bereich des ersten Meters des aktiven Abschnitts des Katalysatorfestbetts (in Strömungsrichtung des Reaktionsgasgemischs). |
| | |
| | Der Außendurchmesser der Thermohülse betrug 10 mm. |
| | |
| | Die Befüllung der Thermorohre erfolgte wie folgt: |
| | |
| | Ein Thermorohr wurde mit dem hergestellten ringförmigen Schalenkatalysator befüllt. Zusätzlich wurden in das Thermorohr zwei Geometrien kugelförmiger Schalenkatalysatoren beigefüllt (gleiche Aktivmasse wie der ringförmige Schalenkatalysator, der Durchmesser der zwei Sorten Steatit C220 (CeramTec) Trägerkugeln betrug 2-3 mm und 4 - 5 mm; der Aktivmassenanteil betrug in beiden Fällen 20 Gew.-%, die Herstellung erfolgte wie bei dem ringförmigen Schalenkatalysator beschrieben, Bindemittel war jedoch eine entsprechende Menge Wasser). |
| | |
| | Die Befüllung der kugelförmigen Schalenkatalysatoren erfolgte über den gesamten aktiven Abschnitt des Katalysatorfestbetts des jeweiligen Thermorohrs homogen verteilt so, dass der Druckverlust des Reaktionsgasgemischs beim Durchgang durch das Thermorohr demjenigen beim Durchgang des Reaktionsgasgemischs durch ein Arbeitsrohr entsprach (bezogen auf den aktiven Abschnitt des Katalysatorfestbetts (d.h., die Inertabschnitte ausgenommen) im Thermorohr waren dazu insgesamt 5 bis 40 Gew.-% an den kugelförmigen Schalenkatalysatoren erforderlich). Gleichzeitig war die jeweilige Gesamtfüllhöhe von Aktiv- und Inertabschnitten in den Arbeits- und Thermorohren gleich bemessen und das Verhältnis von im Rohr enthaltener Gesamtmenge an Aktivmasse zu Wärmeübergangsfläche des Rohres bei Arbeits- und Thermorohren auf denselben Wert eingestellt. |
| | Das in der zweiten Reaktionsstufe erhaltene Produktgasgemisch 2 wurde durch die untere Reaktorhaube des Rohrbündelreaktors heraus- und seiner Aufarbeitung zugeführt. |
| | |
| | Die Umsatzkontrolle und -einstellung in den beiden Reaktionsstufen erfolgte an Hand der Propylen- bzw. Acroleinrestgehalte im Produktgemisch 2. |

L1 wurde zu 130 NI/l• gewählt. Die Zusammensetzung des Reaktionsgaseingangsgemischs war:

| | |
|---|---|
| 6,0 Vol.-% | Propylen, |
| 10,4 Vol.-% | O₂, |
| 1,4 Vol.-% | H₂O, |
| 0,4 Vol.-% | CO, |
| 0,9 Vol.-% | CO₂ und |
| 80,9 Vol.-% | N₂. |

T_{w}^{1, ein} betrug 328 °C. T_{w}^{2,ein} war 270 °C. T_{G}^{E*,1} war 300 °C und T_{G}^{E,2} war 240 °C. L² war 93 NI/l•h. Der Propylenumsatz U^{P} betrug 95 mol-% (bezogen auf den einmaligen Durchgang des Reaktionsgasgemischs durch beide Reaktionsstufen) und der Acroleinumsatz U^{Ac} war 99,4 mol-% (ebenfalls bezogen auf einen einmaligen Durchgang des Reaktionsgasgemischs). Die Selektivität der Acrylsäurebildung S^{AS} betrug 92 mol-% bezogen auf umgesetztes Propylen.

Diese zweistufige Gasphasenpartialoxidation wurde unter im wesentlichen gleichbleibenden Bedingungen (T_{w}^{1,ein} und T_{w}^{2,ein} wurden sukzessive erhöht, um U^{P} und U^{Ac} aufrechtzuerhalten; gemäß WO 2005/042459 wurden Zwischenregenierungen der Katalysatorfestbetten durchgeführt) über einen Zeitraum von vier Jahren betrieben. T_{w}^{1,ein} betrug nach vier Jahren Betriebsdauer 348 °C und T_{w}^{2,ein} betrug 299 °C.

Dann wurde die Partialoxdiation unterbrochen und in beiden Reaktionsstufen das Katalysatorfestbett entnommen.

Mit Hilfe einer kreiszylindrischen Rohrbürste mit spiralförmig ausgeführtem Besatz (mit Besatz versehene Zylinderlänge = 100 mm; Außendurchmesser der Rohrbürste = 24 mm; Borstenlänge = 8,5 mm; Borstenmaterial = DIN Werkstoff 1.4571; Borstendicke = 0,2 mm;) wurden sämtliche Reaktionsrohre der beiden Rohrbündelreaktoren durch Bürsten gereinigt.

Die Analyse (Atomemissionsspektroskopie mit induktiv gekoppeltem Plasma) des aus den Reaktionsrohren der ersten Reaktionsstufe abgebürsteten, auf den Reaktionsrohrinnenwänden befindlichen, Belags ergab für die von Sauerstoff verschiedenen Elemente die nachfolgenden Gewichtsanteile:

| | |
|---|---|
| C | < 0,5 Gew.-%; |
| Co | < 0,32 Gew.-%; |
| Fe | 0,17 Gew.-%; |
| Mo | 65 Gew.-%. |

Der Glühverlust (3 h bei 600 °C an Luft) des abgebürsteten Belagmaterials betrug 0,5 Gew.-%.
Gerechnet als MoO₃ waren 97,5 Gew.-% des Belags in den Rohrinnenwänden Molybdänoxid.

Eine endoskopische Inspektion der Reaktionsrohre wies deren völlige Belagsfreiheit der Rohrinnenoberfläche nach beendete Bürstung aus.

Die Rohre der zum Zweck der Zwischenkühlung eingesetzten Rohrbündelwärmeaustauschers wurden in analoger Weise durch Bürsten gereinigt wie die Reaktionsrohre.

Eine Wiederbeschickung der beiden Rohrbündelreaktoren nach der durchgeführten Bürstung mit den gleichen, aber frischen Katalysatorfestbetten ergab nach beendeter Formierung der Katalysatorfestbetten die identischen Betriebsdaten wie nach der vorbeschriebenen Erstbeschickung der beiden hintereinandergeschalteten Rohrbündelreaktoren.

### Beispiel 2

Es wurde wie im Beispiel 1 verfahren. Der Innendurchmesser der Reaktionsrohre der ersten Reaktionsstufe war jedoch 21 mm, ihre Anzahl betrug 22087 und der Reaktoraußendurchmesser war 5100 mm. Ferner wurde im Rohrbündelreaktor der ersten Reaktionsstufe die Salzschmelze im einfachen Gegenstrom zum Reaktionsgas in den Reaktionsrohren geführt. Die Reaktionseinbauten waren dementsprechend angepasst.

Die Katalysatorbeschickung bestand auf einer Länge von 270 cm ausschließlich aus dem ringförmigen Vollkatalysator 5x3x2 mm. Der Rohrbündelreaktor der ersten Reaktionsstufe enthält ferner zwei Sorten von Thermorohren (jeweils in einer Anzahl von fünf Stück). Der Innendruchmesser der einen Sorte betrug 25 mm (Außendurchmesser der Thermohülse = 8 mm) und der Innendurchmesser der anderen Sorte betrug 22,8 mm (Außendurchmesser der Thermohülse = 6 mm). In entsprechender Weise betrug die Anzahl der Reaktionsrohre in der zweiten Reaktionsstufe nur 15649 bei einem Reaktionsrohrinnendurchmesser von 25 mm. Der Außendurchmesser des Zweitstufenrohrbündelreaktors war ebenfalls 5100 mm. Die Thermorohre entsprachen jenen in der zweiten Reaktionsstufe von Beispiel 1. Auch im Zweitsstufenrohrbündelreaktor wurde die Salzschmelze im einfachen Gegenstrom zum Reaktionsgas geführt. Die Katalysatorbeschickung war wie im Beispiel 1. Im übrigen wurde im wesentlichen wie im Beispiel 1 verfahren. D. h., nach Unterbrechung der Partialoxidation wurde in beiden Reaktionsstufen das Katalysatorfestbett entnommen. Die Reaktionsrohre der zweiten Reaktionsstufe wurden anschließend mit dem im Beispiel 1 verwendeten Bürstentyp gebürstet. Zum Zweck der Bürstung der Reaktionsrohre der ersten Reaktionsstufe wurde hingegen eine kreiszylindrische Rohrbürste mit spiralförmig ausgeführtem Besatz der Firma Hommel Hercules Werkzeughandel, Heidelbergerstraße 52, D-68519 Viernheim verwendet (Außendurchmesser der Bürste = 21 mm, mit Besatz versehene Zylinderlänge = 100 mm). Die Bezeichnung der Bürste war:

Rohrbürste 6202 - 808525 Durchmesser 21x100x160mm, Gewinde 12" BSW, gewellter Messingdraht des Durchmessers 0,2 mm als Besatz, Einzelspirale.

Alternativ kann hier auch eine entsprechende Rohrbürste mit Edelstahlborsten verwendet werden, deren Außendurchmesser lediglich 20,8 mm beträgt.

US Provisional Patent Application No. 60/941385, eingereicht am 01.06.07, ist eingefügt in die vorliegende Anmeldung durch Literaturhinweis.
Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

## Patentansprüche

1. Verfahren der Wiederbeschickung der Reaktionsrohre eines Rohrbündelreaktors mit einem neuen Katalysatorfestbett zum Zweck der Durchführung einer neuen heterogen katalysierten partiellen Gasphasenoxidation einer organischen Ausgangsverbindung in dem neuen Katalysatorfestbett, bei dem vor der Wiederbeschickung der Reaktionsrohre des Rohrbündelreaktors mit dem neuen Katalysatorfestbett in den selben Reaktionsrohren eine heterogen katalysierte partielle Vorgängergasphasenoxidation einer organischen Verbindung in einem in den Reaktionsrohren befindlichen Vorgängerkatalysatorfestbett, das wenigstens Katalysatorformkörper umfasste, deren Aktivmasse ein das Element Mo in oxidiertem Zustand enthaltendes Multielementoxid ist, unter Erzeugung eines Wasserdampf enthaltenden Produktgasgemischs durchgeführt worden und das Vorgängerkatalysatorfestbett nach Beendigung dieser Partialoxidation aus den Reaktionsrohren entnommen worden ist, **dadurch gekennzeichnet, dass** zwischen der Entnahme des Vorgängerkatalysatorfestbetts aus den Reaktionsrohren und der Wiederbeschickung dieser Reaktionsrohre mit dem neuen Katalysatorfestbett, wenigstens bei einem Teil der Reaktionsrohre ein auf deren Innenwand abgeschiedener, Molybdänoxid und/oder Molybdänoxidhydrat enthaltender Feststofffilm (Feststoffbelag) mit Hilfe einer Bürste wenigstens teilweise weggebürstet wird.

2. Verfahren nach Anspruch 1, **dadurch gekennzeichnet, dass** als Bürste eine kreiszylindrische Rohrbürste verwendet wird.

3. Verfahren nach Anspruch 1, **dadurch gekennzeichnet, dass** als Bürste eine kreiszylindrische Rohrbürste mit spiralförmig ausgeführtem Besatz verwendet wird.

4. Verfahren nach einem der Ansprüche 1 bis 3, **dadurch gekennzeichnet, dass** der von der Innenwand der Reaktionsrohre weggebürstete Feststoffbelag unmittelbar bei seiner Entstehung aus den Reaktionsrohren abgesaugt wird.

5. Verfahren nach einem der Ansprüche 1 bis 4, **dadurch gekennzeichnet, dass** die heterogen katalysierte partielle Vorgängergasphasenoxidation eine solche von Propylen zu Acrolein und/oder Acrylsäure, oder von Acrolein zu Acrylsäure, oder von iso-Buten zu Methacrolein und/oder Methacrylsäure, oder von Methacrolein zu Methacrylsäure, oder von Propan zu Acrolein und/oder Acrylsäure, oder von iso-Butan zu Methacrolein und/oder Methacrylsäure, oder von Propylen zu Acrylnitril, oder von Propan zu Acrylnitril, oder von iso-Buten zu Methacrylnitril, oder von iso-Butan zu Methacrylnitril, oder von einem oder mehreren C₄-Kohlenwasserstoffen zu Maleinsäureanhydrid oder von Methanol zu Formaldehyd ist.

6. Verfahren gemäß einem der Ansprüche 1 bis 5, **dadurch gekennzeichnet, dass** sich an das Verfahren der Wiederbeschickung ein Verfahren der heterogen katalysierten partiellen Gasphasenoxidation einer organischen Ausgangsverbindung in dem wiederbeschickten Rohrbündelreaktor anschließt.

7. Verfahren zur Nachsorge einer heterogen katalysierten partiellen Gasphasenoxidation einer organischen Verbindung, die im in den Reaktionsrohren eines Rohrbündelreaktors befindlichen Katalysatorfestbett durchgeführt worden ist, wobei das Katalysatorfestbett wenigstens Katalysatorformkörper umfasste, deren Aktivmasse ein das Element Mo in oxidiertern Zustand enthaltendes Multielementoxid ist und ein Wasserdampf enthaltendes Produktgasgemisch erzeugt wurde, **dadurch gekennzeichnet, dass** man das Katalysatorfestbett aus den Reaktionsrohren des Rohrbündelreaktors entnimmt und wenigstens bei einem Teil der Reaktionsrohre den auf deren Innenwand abgeschiedenen, Molybdänoxid und/oder Molybdänoxidhydrat enthaltenden, Feststoffbelag mit Hilfe einer Bürste wenigstens teilweise wegbürstet.

## Claims

1. A process for recharging the reaction tubes of a tube bundle reactor with a new fixed catalyst bed for the purpose of performing a new heterogeneously catalyzed partial gas phase oxidation of an organic starting compound in the new fixed catalyst bed, in which, before the recharge of the reaction tubes of the tube bundle reactor with the new fixed catalyst bed in the same reaction tubes, a heterogeneously catalyzed partial preceding gas phase oxidation of an organic compound has been performed in a preceding fixed catalyst bed which was disposed in the reaction tubes and comprised at least shaped catalyst bodies whose active composition is a multielement oxide comprising the element Mo in the oxidized state to obtain a steam-comprising product gas mixture, and the preceding fixed catalyst bed, after this partial oxidation has ended, has been withdrawn from the reaction tubes, wherein, between the withdrawal of the preceding fixed catalyst bed from the reaction tubes and the recharge of these reaction tubes with the new fixed catalyst bed, at least in some of the reaction tubes, a solid film (solid deposit) which comprises molybdenum oxide and/or molybdenum oxide hydrate and has been deposited on their inner wall is brushed away at least partly with the aid of a brush.

2. The process according to claim 1, wherein the brush used is a cylindrical tube brush.

3. The process according to claim 1, wherein the brush used is a cylindrical tube brush with a spiral head.

4. The process according to any of claims 1 to 3, wherein the solid deposit brushed away from the inner wall of the reaction tubes is sucked out of the reaction tubes as soon as it is formed.

5. The process according to any of claims 1 to 4, wherein the heterogeneously catalyzed partial preceding gas phase oxidation is one of propylene to acrolein and/or acrylic acid, or of acrolein to acrylic acid, or of isobutene to methacrolein and/or methacrylic acid, or of methacrolein to methacrylic acid, or of propane to acrolein and/or acrylic acid, or of isobutane to methacrolein and/or methacrylic acid, or of propylene to acrylonitrile, or of propane to acrylonitrile, or of isobutene to methacrylonitrile, or of isobutane to methacrylonitrile, or of one or more C₄ hydrocarbons to maleic anhydride, or of methanol to formaldehyde.

6. The process according to any of claims 1 to 5, wherein the process for recharging is followed by a process for heterogeneously catalyzed partial gas phase oxidation of an organic starting compound in the recharged tube bundle reactor.

7. A process for maintaining a heterogeneously catalyzed partial gas phase oxidation of an organic compound which has been performed in the fixed catalyst bed disposed in the reaction tubes of a tube bundle reactor, the fixed catalyst bed having comprised at least shaped catalyst bodies whose active composition is a multielement oxide comprising the element Mo in the oxidized state and a steam-comprising product gas mixture having been obtained, wherein the fixed catalyst bed is removed from the reaction tubes of the tube bundle reactor and, in at least some of the reaction tubes, the solid deposit which comprises molybdenum oxide and/or molybdenum oxide hydrate and has been deposited on their inner wall is brushed away at least partly with the aid of a brush.

## Revendications

1. Procédé de rechargement des tubes de réaction d'un réacteur à faisceau de tubes avec un nouveau lit catalytique fixe pour la réalisation d'une nouvelle oxydation partielle en phase gazeuse sous catalyse hétérogène d'un composé de départ organique dans le nouveau lit catalytique fixe, selon lequel une oxydation partielle en phase gazeuse sous catalyse hétérogène précédente d'un composé organique dans un lit catalytique fixe précédent se trouvant dans les tubes de réaction, qui comprend au moins des corps moulés catalytiques dont la masse active est un oxyde de plusieurs éléments contenant l'élément Mo à l'état oxydé, pour former un mélange gazeux de produits contenant de la vapeur d'eau a été réalisée dans les mêmes tubes de réaction avant le rechargement des tubes de réaction du réacteur à faisceau de tubes avec le nouveau lit catalytique fixe, et le lit catalytique fixe précédent a été extrait des tubes de réaction à la fin de cette oxydation partielle, **caractérisé en ce qu'**entre l'extraction du lit catalytique fixe précédent des tubes de réaction et le rechargement de ces tubes de réaction avec le nouveau lit catalytique fixe, pour au moins une partie des tubes de réaction, un film solide (dépôt solide) contenant de l'oxyde de molybdène et/ou un hydrate d'oxyde de molybdène déposé sur leur paroi intérieure est au moins partiellement éliminé par brossage à l'aide d'une brosse.

2. Procédé selon la revendication 1, **caractérisé en ce qu'**une brosse tubulaire cylindrique est utilisée en tant que brosse.

3. Procédé selon la revendication 1, **caractérisé en ce qu'**une brosse tubulaire cylindrique à garniture agencée en spirale est utilisée en tant que brosse.

4. Procédé selon l'une quelconque des revendications 1 à 3, **caractérisé en ce que** le dépôt solide éliminé par brossage de la paroi intérieure des tubes de réaction est aspiré hors des tubes de réaction dès sa formation.

5. Procédé selon l'une quelconque des revendications 1 à 4, **caractérisé en ce que** l'oxydation partielle en phase gazeuse sous catalyse hétérogène précédente est une telle oxydation de propylène en acroléine et/ou acide acrylique ou d'acroléine en acide acrylique ou d'iso-butène en méthacroléine et/ou acide méthacrylique ou de méthacroléine en acide méthacrylique ou de propane en acroléine et/ou acide acrylique ou d'iso-butane en méthacroléine et/ou acide méthacrylique ou de propylène en acrylonitrile ou de propane en acrylonitrile ou d'iso-butène en méthacrylonitrile ou d'iso-butane en méthacrylonitrile ou d'un ou de plusieurs hydrocarbures en C₄ en anhydride de l'acide maléique ou de méthanol en formaldéhyde.

6. Procédé selon l'une quelconque des revendications 1 à 5, **caractérisé en ce qu'**un procédé d'oxydation partielle en phase gazeuse sous catalyse hétérogène d'un composé de départ organique dans le réacteur à faisceau de tubes rechargé suit le procédé de rechargement.

7. Procédé de maintenance d'une oxydation partielle en phase gazeuse sous catalyse hétérogène d'un composé organique, qui a été réalisée dans le lit catalytique fixe se trouvant dans les tubes de réaction d'un réacteur à faisceau de tubes, le lit catalytique fixe comprenant au moins des corps moulés catalytiques dont la masse active est un oxyde de plusieurs éléments contenant l'élément Mo à l'état oxydé et un mélange gazeux de produits contenant de la vapeur d'eau ayant été généré, **caractérisé en ce que** le lit catalytique fixe est extrait des tubes de réaction du réacteur à faisceau de tubes et, pour au moins une partie des tubes de réaction, le dépôt solide contenant de l'oxyde de molybdène et/ou un hydrate d'oxyde de molybdène déposé sur leur paroi intérieure est au moins partiellement éliminé par brossage à l'aide d'une brosse.
